# EUROPÄISCHE PATENTANMELDUNG

(11) **EP 2 893 947 A1**
(43) Veröffentlichungstag der Anmeldung: **15.07.2015**
(21) Anmeldenummer: 14150716.0
(22) Anmeldetag: 10.01.2014
(51) Int. Cl.: A61M 11/06, A61M 11/08, A61M 15/00, A61M 39/24, B05B 11/00, B05B 11/02, B65D 39/00, B65D 39/08, B65D 39/16, B65D 43/02, B65D 43/06, B65D 43/12, B65D 83/20, B65D 83/40, B65D 50/04, B65D 50/06

(54) **Zerstäuber**

(71) Anmelder: BOEHRINGER INGELHEIM INTERNATIONAL GMBH, 55216 Ingelheim am Rhein (DE)
(72) Erfinder: Die Erfindernennung liegt noch nicht vor
(74) Vertreter: Simon, Elke Anna Maria

(57) **Zusammenfassung**

Die Erfindung betrifft einen Zerstäuber (1) für ein Fluid, in den ein Behälter mit dem Fluid einsetzbar ist, mit einem Druckerzeuger zur Förderung und/oder Zerstäubung des Fluids, mit einer Zugangsöffnung (Z) zum Einsetzen des Behälters und mit Befestigungsmitteln zur lösbaren Befestigung eines Gehäuseunterteils.

Zur Erhöhung der Flexibilität während der Produktion und auch hinsichtlich des Vertriebs des Zerstäubers (1) mitsamt Behälter wird erfindungsgemäß vorgeschlagen, dass die Zugangsöffnung (Z) durch ein lösbares Verschlusselement (25) verschlossen ist.

## Beschreibung

Die vorliegende Erfindung betrifft einen Zerstäuber gemäß dem Oberbegriff des Anspruchs 1.

Ausgangspunkt der vorliegenden Erfindung ist ein Zerstäuber in Form eines Inhalators, wie im Grundprinzip in der WO 97/12687 A1 (Fig. 6a, 6b) und in konkreter Ausgestaltung in der WO 09/047173 sowie in den Fig. 1 und 2 der hier zugehörigen Zeichnung dargestellt. Der Zerstäuber weist als Reservoir für ein zu zerstäubendes Fluid einen einsetzbaren Behälter mit dem Fluid und einen Druckerzeuger mit einer Antriebsfeder zur Förderung und Zerstäubung des Fluids auf.

Zur Vervollständigung der Offenbarung der vorliegenden Patentanmeldung wird vorsorglich auf den kompletten Offenbarungsgehalt sowohl der WO 97/12687 A1 als auch der WO 09/047173 verwiesen. Generell bezieht sich die dortige Offenbarung bevorzugt auf einen Zerstäuber mit einem Federdruck von 5 bis 60 mPa, bevorzugt 10 bis 50 mPa auf das Fluid, mit Volumina pro Hub von 10 bis 50 µl, bevorzugt 10 bis 20 µl, ganz bevorzugt etwa 15 µl pro Hub, Teilchengrößen von bis zu 20 µm, bevorzugt 3 bis 10 µm.

Ferner bezieht sich die dortige Offenbarung bevorzugt auf einen Zerstäuber mit zylinderähnlicher Form und einer Größe von etwa 9 cm bis etwa 15 cm in der Länge und etwa 2 cm bis etwa 5 cm in der Breite sowie von einer Düsen-Streiffächerung von 20° bis 160°, bevorzugt von 80° bis 100°. Derartige Werte gelten auch für den Zerstäuber nach der Lehre der Erfindung als besonders bevorzugte Werte.

Durch Drehen eines Betätigungsteils in Form eines Gehäuseteils des Zerstäubers ist die Antriebsfeder spannbar und Fluid in eine Druckkammer des Druckerzeugers saugbar. Nach manueller Betätigung eines Sperrelements wird das Fluid in der Druckkammer von der Antriebsfeder unter Druck gesetzt und zerstäubt, also unter Bildung eines Aerosols ausgegeben. Beim Spannen einerseits und der dann folgenden Zerstäubung andererseits führt der Behälter jeweils eine Hubbewegung aus.

Der Zerstäuber weist eine mechanische Überwachungseinrichtung auf, die zur Zählung von Betätigungen des Zerstäubers das Drehen des Betätigungsteils erfasst.

Bei den bekannten Zerstäubern können Behälter mit unterschiedlichen Fluiden, also insbesondere unterschiedlichen Arzneimitteln bzw. Wirkstoffen, eingesetzt werden. Um einen solchen Zerstäuber und einen Behälter für einen solchen Zerstäuber mit verbesserter Sicherheit gegen Verwechslung des Behälters bei der Benutzung bereitzustellen, ist aus der WO 2005/080002 A1 ein verbesserter Zerstäuber und ein verbesserter Behälter vorgestellt worden.

Dessen grundlegende Verbesserung bestand darin, eine Kodierung vorzusehen, so dass nur ein bestimmter Behälter oder mehrere bestimmte Behälter mit einem dafür vorgesehenen Zerstäuber verwendbar, insbesondere in diesen einsetzbar ist bzw. sind. Hierfür weist der Zerstäuber ein erstes Kodiermittel auf, dem Behälter ist ein zweites Kodiermittel zugeordnet. Die Kodiermittel wirken derart zusammen, dass der Behälter mit dem zugeordneten zweiten Kodiermittel nur dann in den Zerstäuber einsetzbar oder mit diesem verwendbar ist, wenn die Kodiermittel zueinander passen bzw. eine passende Kodierung bilden.

Die bekannten Zerstäuber, wie auch der Zerstäuber nach der vorliegenden Erfindung arbeiten ausschließlich mechanisch, d. h. ohne Treibgas und ohne Elektrik.

Das Innere des Zerstäubers, insbesondere der Druckerzeuger mitsamt einem nadelartigen Förderrohrs, welches eine Kapillare zur Förderung des Fluids umfasst, ist sehr empfindlich gegen Verschmutzung. Daher werden nach dem Stand der Technik die Zerstäuber so früh wie möglich mit dem Gehäuseunterteil zusammengebracht und auch zusammen mit diesem vertrieben. Dies führt nach derzeitigen Erkenntnissen jedoch zu einer ungebührlichen Einschränkung der Flexibilität.

Der vorliegenden Erfindung liegt daher die Aufgabe zugrunde, einen bekannten Zerstäuber derart zu verbessern, dass hier Abhilfe geschaffen wird.

Die vorliegende Aufgabe wird mit den kennzeichnenden Merkmalen von Patentanspruch 1 gelöst. Vorteilhafte Ausführungen beziehungsweise Weiterbildungen der Erfindung sind den jeweiligen Unteransprüchen entnehmbar.

Die Erfindung geht daher zunächst aus von einem Zerstäuber für ein Fluid, in den ein Behälter mit dem Fluid einsetzbar ist, mit einem Druckerzeuger zur Förderung und/oder Zerstäubung des Fluids, mit einer Zugangsöffnung zum Einsetzen des Behälters und mit Befestigungsmitteln zur lösbaren Befestigung eines Gehäuseunterteils,

Erfindungsgemäß ist nun vorgesehen, dass die Zugangsöffnung durch ein lösbares Verschlusselement verschlossen ist.

Die vorschlagsgemäße Lösung führt zu einer deutlichen Erhöhung der Flexibilität. Auf diese Weise ist es unerheblich, ob vor, während oder auch nach dem Produktionsprozess (z.B. beim Patienten) der Zerstäuber vom Gehäuseunterteil und auch von dem das zu zerstäubende Fluid enthaltenden Behälter getrennt ist. Das Innere des Zerstäubers ist zu jedem Zeitpunkt geschützt. Dies führt zu einer deutlichen Erhöhung der Flexibilität hinsichtlich der Fertigung/Montage. Der Zerstäuber und ein passendes Gehäuseunterteil können ohne Risiko völlig getrennt voneinander gefertigt, transportiert oder gelagert werden. Auch ist es nicht mehr zwingend, den Zerstäuber als Verkaufseinheit mit bereits montiertem Gehäuseunterteil in den Verkehr zu bringen.

Vorteile ergeben sich insbesondere auch bei der Verwendung verschiedener Wirkstoffe. So ist es beispielsweise möglich, mehrere passende Gehäuseunterteile fest mit verschiedenen Wirkstoff-Behältern (Kartuschen) zu bestücken. Die Verwendungsentscheidung für den Zerstäuber muss dann nicht bereits bei der Zerstäuberfertigung getroffen werden, sondern kann auf einen späteren Zeitpunkt verschoben werden.

Gemäß einer ersten vorteilhaften Weiterbildung der Erfindung ist vorgesehen, dass sich das Verschlusselement klemmend und/oder rastend und/oder gewindeartig am Zerstäuber festhält. Auf diese Weise ist ein guter und damit sicherer Halt des Verschlusselementes am Zerstäuber gewährleistet. Beispielsweise kann das Verschlusselement kappenartig ausgebildet sein und den Zerstäuber bzw. ein mit diesem verbundenes, ringartiges Teil radial von außen übergreifen.

Es ist gemäß einer sehr vorteilhaften Ausbildung der Erfindung aber auch denkbar, dass sich das Verschlusselement klemmend und/oder rastend und/oder gewindeartig im Innern des Zerstäubers festhält. Das Verschlusselement ist dabei also nach Art eines Stopfens ausgebildet, wobei das Innere des Zerstäubers optimal zur Befestigung des Verschlusselementes genutzt und dadurch auch gleichzeitig noch besser geschützt werden kann.

Gemäß einer anderen Ausbildung der Erfindung kann sich das Verschlusselement klemmend und/oder rastend und/oder gewindeartig an einem Verschlussring festhalten, welcher als unterer Anschlag für eine zum Druckerzeuger gehörige Antriebsfeder dient.

Hierdurch lässt sich auf einfache Weise eine höchst stabile Befestigung des Verschlusselementes an einem ohnehin vorhandenen Bauteil des Zerstäubers realisieren.

Alternativ ist es möglich, dass sich das Verschlusselement rastend, klemmend oder gewindeartig an einer zum Druckerzeuger gehörigen Antriebsfeder festhält. Hierdurch lässt sich auf einfache Weise ebenso ein sicherer und zuverlässiger Halt des Verschlusselementes erzielen.

Das Verschlusselement kann zweckmäßigerweise eine kreisringförmige Wandung aufweisen, an der radial außenseitig wenigstens zwei Befestigungselemente angeordnet sind, welche sich klemmend und/oder rastend im Bereich der Zugangsöffnung festhalten. Besonders bevorzugt sind dabei die Befestigungselemente als an die kreisringförmige Wandung angeformte, stufenartige Elemente ausgebildet, mit wenigstens zwei in Einsteckrichtung des Verschlusselementes radial nach außen ansteigenden Stufen.

Alternativ ist vorstellbar, die Befestigungselemente als an die kreisringförmige Wandung außenseitig angeformte, parallel zu einer Einsteckrichtung des Verschlusselementes ausgerichtete Stege auszubilden.

In beiden Fällen ist eine Montage und Demontage des Verschlusselementes sehr leicht möglich, wobei dennoch ein sicherer Halt des Verschlusselementes am Zerstäuber gewährleistet ist.

Gemäß einer anderen Weiterbildung der Erfindung wird vorgeschlagen, das Verschlusselement mit dem Zerstäuber zu verkleben. Dies kann vorzugsweise nach Art einer Siegelfolie oder beispielsweise mittels eines Zwei-Komponenten (2K)-Aufbaus erfolgen.

Die Siegelfolie ist bevorzugt mit dem verdrehbaren Innenteil des Zerstäubers oder mit einem wiederum an diesem Innenteil gehaltenen, ring- oder hülsenartigen Kodierelement verklebt.

Der erwähnte 2K-Aufbau kann ein kreisrundes Pappelement umfassen, welches randseitig in die Zugangsöffnung eingeklebt ist und mit dem unterseitig eine Kunststofflasche zum Abziehen des Verschlusselementes fest verbunden ist.

Eine einfach handhabbare Befestigung kann auch über eine bajonettartige Verbindung zwischen Zerstäuber und Verschlusselement bewerkstelligt werden. Hierbei wird das Verschlusselement also zunächst auf den Zerstäuber aufgeschoben und anschließend durch Verdrehen gesichert.

Es ist höchst vorteilhaft, wenn das Verschlusselement mit einer planen Standfläche versehen ist, denn auf diese Weise kann der Zerstäuber mitsamt Verschlusselement bei Bedarf jederzeit sicher aufgestellt werden.

Zweckmäßigerweise weist das Verschlusselement im Bereich der Standfläche wenigstens eine schlitzartige Materialausnehmung auf, derart, dass dadurch wenigstens ein ausklappbares Greifelement ausgebildet ist.

Hierdurch wird ein Lösen des Verschlusselementes enorm erleichtert.

Besonders vorteilhaft ist die Materialausnehmung teilkreisartig, insbesondere halbkreisartig ausgebildet. Dadurch kann beim Herausklappen des Greifelementes eine Art Halbring gebildet werden, in den ein Finger steckbar und somit eine gute Kraftübertragung zum Herausziehen möglich ist.

Wenn zwei halbkreisartige Materialausnehmungen vorhanden sind, kann eine solche Kraftübertragung optimiert werden.

Die Erfindung betrifft unabhängig vom Verschlusselement auch eine Montageeinheit, bestehend aus einem erfindungsgemäßen Zerstäuber und wenigstens einem mit dem Zerstäuber lösbar verbindbaren, gehäuseartigen Teil, an dem der Behälter gehalten ist. Der Behälter ist vorzugsweise unlösbar am gehäuseartigen Teil gehalten, das heißt nicht zerstörungsfrei oder zumindest nur mit einem Spezialwerkzeug lösbar.

Eine solche Montageeinheit bietet einem Kunden die bequeme Möglichkeit, gleich "auf Vorrat" zu kaufen. Aufgrund des vorhandenen, lösbaren Verschlusselementes am Zerstäuber kann bereits bei einer Erstmontage eines der passenden Gehäuseunterteile die Gefahr einer Verschmutzung des Zerstäubers deutlich verringert werden. Der Kunde braucht das Verschlusselement nämlich erst dann zu entfernen, wenn er sich nach dem Öffnen der verpackten Montageeinheit über das zu montierende Gehäuseunterteil und die Art der zu erfolgenden Montage Klarheit verschafft hat.

Entsprechend liegt der Vorteil auch bei einem erforderlichen Austausch des Gehäuseunterteils aufgrund verbrauchten Wirkstoffs im Behälter. Der Kunde kann nach Entfernen des alten Gehäuseunterteils vom Zerstäuber vorübergehend das ursprünglich vorhandene Verschlusselement wieder verwenden und ohne die Gefahr einer Verschmutzung die Auswahl für das nächste zu montierende Gehäuseunterteil treffen.

Die erfindungsgemäße Montageeinheit kann zweckmäßigerweise derart weitergebildet werden, dass Kodiermittel am Zerstäuber und am gehäuseartigen Teil vorhanden sind, derart, dass das gehäuseartige Teil mitsamt Behälter nur dann in den Zerstäuber einsetzbar oder mit diesem verwendbar ist, wenn die Kodiermittel eine zueinander passende Kodierung aufweisen.

Auf diese Weise können ein oder mehrere ein bestimmtes Medikament bzw. einen bestimmten Wirkstoff enthaltende Behälter zusammen mit dem Zerstäuber als eindeutig kodierte, unverwechselbare Verkaufseinheit angeboten werden.

Vorzugsweise ist dabei wenigstens eines der Kodiermittel in unterschiedlichen, definierten Positionen an den Zerstäuber montierbar und wenigstens ein anderes der Kodiermittel in unterschiedlichen, definierten Positionen oder zumindest austauschbar an dem lösbaren Gehäuseteil.

Somit kann auch das gehäuseartige Bauteil mit nur einem Kodiermittel an unterschiedliche Kodierungen angepasst werden. Zumindest ist bei einer erforderlichen Änderung der Kodierung nicht der Austausch des gesamten gehäuseartigen Bauteils notwendig, sondern nur des Kodiermittels, so dass hierdurch auch Kostenersparnisse erzielbar sind. Kostenersparnisse lassen sich unter anderem auch dadurch erzielen, dass bei der Fertigung von Geräten mit unterschiedlicher Kodierung keine getrennten Fertigungslinien nötig sind. Es ist lediglich für den Einbau der unterschiedlichen Kodiermittel auf ein und derselben Fertigungslinie Sorge zu tragen.

Die Gehäuseteile des Zerstäubers sowie das lösbare Gehäuseteil sind vorzugsweise aus Kunststoff im Spritzgussverfahren hergestellt. Bevorzugt ist hierfür Polybutylenterephthalat (PBT) oder auch Acrylnitril-Butadien-Styrol-Copolymerisat (ABS) einsetzbar.

Das Verschlusselement kann vorzugsweise aus Kunststoff oder auch aus Verbundmaterialien bestehen. Auch eine folienartige Ausbildung ist, wie bereits erwähnt, denkbar.

Als einzusetzende Kunststoffe sind Polyolefine (Polyethylen, Polypropylen, Polybuten, etc.), Polyester, Vinylpolmer (Polystyrol, Polyvinylchlorid, etc.), Fluoropolymer, Polyamide, Polycarbonate, Polyurethane, Elastomere sowie auch deren silikonisierte Varianten bevorzugt. Das gewählte Material kann farblos oder auch farbig ausgebildet sein.

Zur zuverlässigen Herstellung des Verschlusselementes können verschiedene Verfahren zur Anwendung kommen, wie zum Beispiel Thermoforming (Spritzguss, Tiefziehen, Extrusion, etc.), Schäumen oder auch mechanische Bearbeitungsverfahren.

Bevorzugte Ausführungsbeispiele der Erfindung sind in den Zeichnungen dargestellt und werden in der nachfolgenden Beschreibung näher erläutert. Dabei beziehen sich gleiche Bezugszeichen auf gleiche, vergleichbare oder funktional gleiche Bauteile, wobei entsprechende oder vergleichbare Eigenschaften und Vorteile erreicht werden, auch wenn eine wiederholte Beschreibung weggelassen ist.

Es zeigen, jeweils schematisch
- Fig. 1: einen Längsschnitt eines bekannten Zerstäubers im ungespannten Zustand,
- Fig. 2: einen um 90 Grad gegenüber Fig. 1 gedrehten Längsschnitt des bekannten Zerstäubers im gespannten Zustand,
- Fig. 3: eine perspektivische Darstellung eines bevorzugten Ausführungsbeispiels der Erfindung,
- Fig. 4: einen Längsschnitt gemäß Schnittverlauf IV aus Fig. 3, also mit noch nicht eingesetztem Verschlusselement,
- Fig. 5: eine vergrößerte Darstellung V aus Fig. 4, jedoch mit eingesetztem Verschlusselement,
- Fig. 6: eine perspektivische Draufsicht auf das Verschlusselement,
- Fig. 7: eine Ansicht des Verschlusselementes von oben,
- Fig. 8: eine Ansicht des Verschlusselementes von unten,
- Fig. 9: eine perspektivische Ansicht einer aus dem erfindungsgemäßen Zerstäuber gem. Fig. 3 und aus drei Gehäuseunterteilen mitsamt Behälter bestehenden Montage- bzw. Verkaufseinheit,
- Fig. 10: eine weitere Ausführungsform eines erfindungsgemäßen Zerstäubers,
- Fig. 11: eine Einzeldarstellung des gem. Fig. 10 verwendeten Verschlusselementes von oben,
- Fig. 12: eine Schnittansicht gemäß Schnittverlauf XII aus Fig. 11,
- Fig. 13: eine Ansicht des verwendeten Verschlusselementes von unten gemäß Ansicht XIII aus Fig. 12,
- Fig. 14: ein weiteres, leicht abgewandeltes Ausführungsbeispiel mit doppelter Grifflasche,
- Fig. 15: das Ausführungsbeispiel aus Fig. 14 mit beidseitig heruntergeklappten Grifflaschen,
- Fig. 16: ein weiteres, verwendbares Verschlusselement,
- Fig. 17: eine Teilansicht eines erfindungsgemäßen Zerstäubers mit eingesetztem Verschlusselement gem. Fig. 16 in einer Längsschnittdarstellung,
- Fig. 18: ein weiteres verwendbares Verschlusselement,
- Fig. 19: eine Teilansicht eines erfindungsgemäßen Zerstäubers mit eingesetztem Verschlusselement gem. Fig. 18 in einer Längsschnittdarstellung,
- Fig. 20: ein weiteres verwendbares Verschlusselement,
- Fig. 21: ein weiteres verwendbares Verschlusselement,
- Fig. 22: eine Teilansicht eines erfindungsgemäßen Zerstäubers mit eingesetztem Verschlusselement gem. Fig. 21 in einer Längsschnittdarstellung,
- Fig. 23: eine Teilansicht eines erfindungsgemäßen Zerstäubers mit einem anderen eingesetztem Verschlusselement in einer Längsschnittdarstellung,
- Fig. 24: ein weiteres verwendbares Verschlusselement,
- Fig. 25: eine Teilansicht eines erfindungsgemäßen Zerstäubers mit eingesetztem Verschlusselement gem. Fig. 24 in einer Längsschnittdarstellung,
- Fig. 26: eine Explosionsdarstellung eines weiteren Ausführungsbeispiels eines erfindungsgemäßen Zerstäubers in einer Teilansicht,
- Fig. 27: ein weiteres verwendbares Verschlusselement,
- Fig. 28: eine Teilansicht eines erfindungsgemäßen Zerstäubers mit eingesetztem Verschlusselement gem. Fig. 27 in einer Längsschnittdarstellung,
- Fig. 29: ein weiteres Ausführungsbeispiel für einen erfindungsgemäßen Zerstäuber in einer Längsschnittdarstellung,
- Fig. 30: ein weiteres verwendbares Verschlusselement,
- Fig. 31: eine Teilansicht eines erfindungsgemäßen Zerstäubers mit eingesetztem Verschlusselement gem. Fig. 30 in einer Längsschnittdarstellung,
- Fig. 32: eine perspektivische Ansicht eines weiteren, verwendbaren Verschlusselementes,
- Fig. 33: ein Längsschnitt durch das Verschlusselement aus Fig. 32,
- Fig. 34: eine perspektivische Ansicht eines weiteren Ausführungsbeispiels für einen erfindungsgemäßen Zerstäuber,
- Fig. 35: ein Längsschnitt durch das Ausführungsbeispiel gemäß Schnittverlauf aus Fig. 34,
- Fig. 36: ein weiteres Ausführungsbeispiel für einen erfindungsgemäßen Zerstäuber in einer perspektivischen Ansicht,
- Fig. 37: ein Längsschnitt durch das Ausführungsbeispiel gem. Fig. 36,
- Fig. 38: ein weiteres verwendbares Verschlusselement,
- Fig. 39: eine Teilansicht eines erfindungsgemäßen Zerstäubers mit eingesetztem Verschlusselement gem. Fig. 38 in einer Längsschnittdarstellung,
- Fig. 40: ein weiteres Ausführungsbeispiel eines erfindungsgemäßen Zerstäubers in einer perspektivischen Darstellung,
- Fig. 41: eine Teilansicht des erfindungsgemäßen Zerstäubers gem. Fig. 40 in einer Längsschnittdarstellung,
- Fig. 42: ein weiteres Ausführungsbeispiel eines erfindungsgemäßen Zerstäubers in perspektivischer Ansicht und
- Fig. 43: eine Teilansicht des erfindungsgemäßen Zerstäubers gem. Fig. 42 in einer Längsschnittdarstellung.

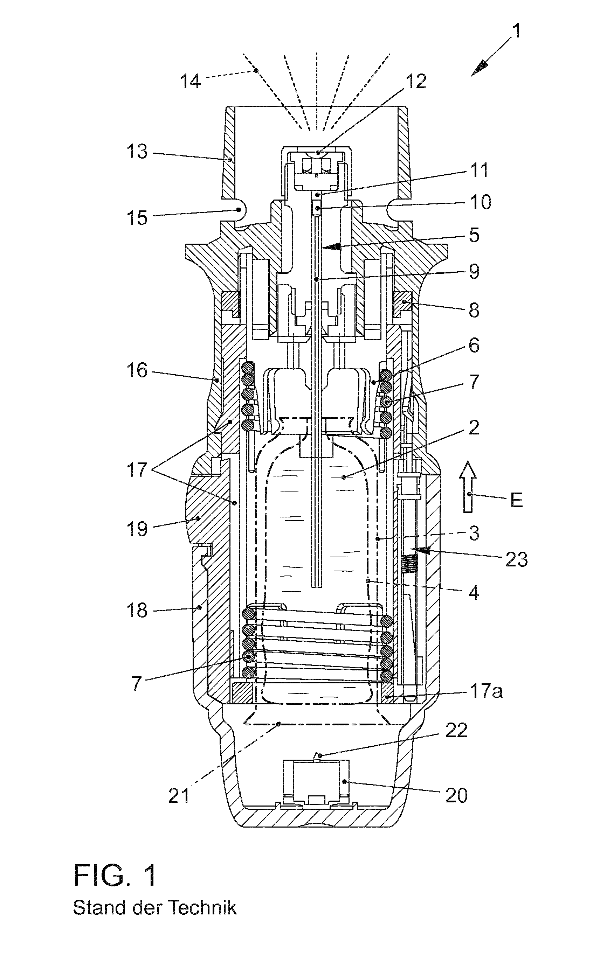

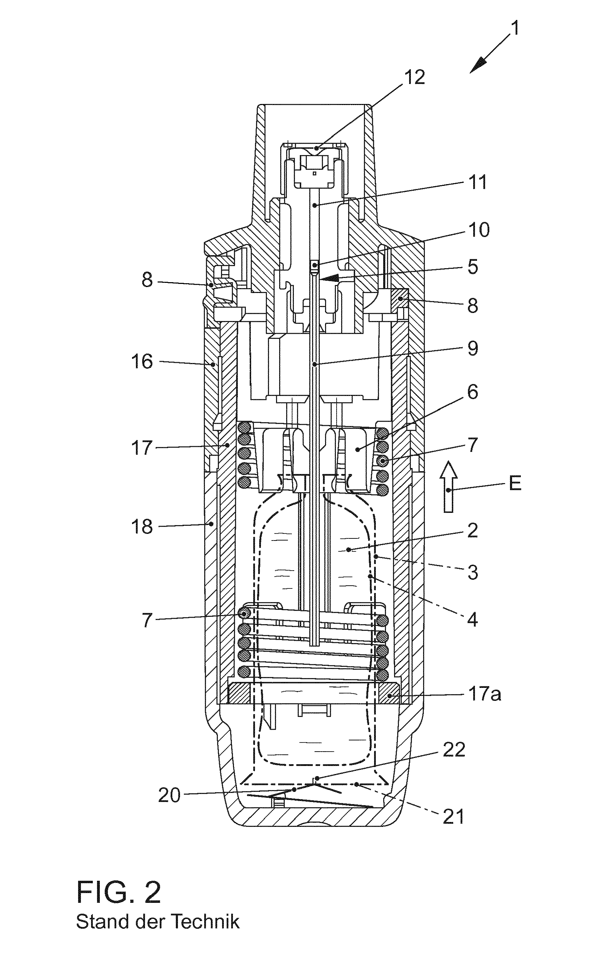

Fig. 1 und 2 zeigen einen bekannten Zerstäuber 1 zur Zerstäubung eines Fluids 2, insbesondere eines hochwirksamen Arzneimittels bzw. Wirkstoffs oder dergleichen, in einer schematischen Darstellung im ungespannten Zustand (Fig. 1) und im gespannten Zustand (Fig. 2). Der Zerstäuber 1 ist insbesondere als tragbarer Inhalator ausgebildet und arbeitet vorzugsweise ohne Treibgas.

Bei Zerstäubung des Fluids 2, vorzugsweise einer Flüssigkeit, insbesondere eines Arzneimittels, wird ein Aerosol gebildet, das von einem nicht dargestellten Benutzer eingeatmet bzw. inhaliert werden kann. Üblicherweise erfolgt das Inhalieren wenigstens einmal täglich, insbesondere mehrmals täglich, vorzugsweise in vorbestimmten Zeitabständen.

Der Zerstäuber 1 weist einen einsetzbaren und vorzugsweise wechselbaren Behälter 3 mit dem Fluid 2 auf, der ein Reservoir für das zu zerstäubende Fluid 2 bildet. Vorzugsweise enthält der Behälter 3 eine ausreichende Menge an Fluid 2 für eine mehrfache Anwendung, insbesondere für eine vorbestimmte Applikationszeit, wie einen Monat, oder für mindestens 50, vorzugsweise mindestens 100 Dosierungen bzw. Zerstäubungen.

Der Behälter 3 ist im Wesentlichen zylindrisch bzw. kartuschenartig ausgebildet und von unten, nach Öffnen des Zerstäubers 1, in diesen einsetzbar und gegebenenfalls wechselbar. Er ist vorzugsweise starr ausgebildet, wobei das Fluid 2 vorzugsweise in einem Beutel 4 im Behälter 3 aufgenommen ist.

Der Zerstäuber 1 weist einen Druckerzeuger 5 zur Förderung und zur Zerstäubung des Fluids 2, insbesondere jeweils in einer vorbestimmten, gegebenenfalls einstellbaren Dosiermenge, auf. Der Druckerzeuger 5 weist eine Halterung 6 für den Behälter 3, eine zugeordnete, nur teilweise dargestellte Antriebsfeder 7 mit einem zur Entsperrung manuell betätigbaren Sperrelement 8, ein Förderrohr 9 mit einem Rückschlagventil 10, eine Druckkammer 11 und eine Austragsdüse 12 im Bereich eines Mundstücks 13 auf.

Beim axialen Spannen der Antriebsfeder 7 wird die Halterung 6 mit dem Behälter 3 und dem Förderrohr 9 bei den Darstellungen nach unten bewegt und Fluid 2 aus dem Behälter 3 in die Druckkammer 11 des Druckerzeugers 5 über das Rückschlagventil 10 gesaugt. Da die Austragsdüse 12 einen sehr geringen Strömungsquerschnitt hat und insbesondere als Kapillare ausgebildet ist, ergibt sich eine so starke Drosselwirkung, dass auch ohne Rückschlagventil an dieser Stelle ein Einsaugen von Luft sicher ausgeschlossen ist.

Beim anschließenden Entspannen nach Betätigung des Sperrelements 8 wird das Fluid 2 in der Druckkammer 11 von der das Förderrohr 9 wieder nach oben bewegenden Antriebsfeder 7 - also durch Federkraft - unter Druck gesetzt und über die Austragsdüse 12 ausgegeben, wobei es zerstäubt wird, insbesondere in Partikel im µm- oder nm-Bereich, vorzugsweise in lungengängige Partikel mit etwa 5 µm, die eine Wolke bzw. einen Strahl eines Aerosols 14 bilden, wie in Fig. 1 angedeutet. Die Förderung und Zerstäubung des Fluids 2 erfolgen vorzugsweise also rein mechanisch, insbesondere ohne Treibgas und ohne Elektrik.

Ein nicht dargestellter Benutzer kann das Aerosol 14 inhalieren, wobei Zuluft über zumindest eine Zuluftöffnung 15 in das Mundstück 13 saugbar ist.

Der Zerstäuber 1 weist ein Gehäuseoberteil 16 und ein dem gegenüber drehbares Innenteil 17 auf, an dem ein insbesondere manuell betätigbares Gehäuseteil 18 vorzugsweise mittels eines Halteelementes 19 lösbar befestigt, insbesondere aufgesteckt ist. Zum Einsetzen und/oder Auswechseln des Behälters 3 ist das Gehäuseteil 18 vom Zerstäuber 1 lösbar.

Durch manuelles Drehen des Gehäuseteils 18 ist das Innenteil 17 relativ zum Gehäuseteil 16 drehbar, wodurch die Antriebsfeder 7 über ein nicht dargestelltes, auf die Halterung 6 wirkendes Getriebe in axialer Richtung spannbar ist. Beim Spannen wird der Behälter 3 axial nach unten bewegt, bis der Behälter 3 eine in Fig. 2 angedeutete Endlage im gespannten Zustand annimmt. Während des Zerstäubungsvorgangs wird der Behälter 3 von der Antriebsfeder 7 wieder in seine Ausgangslage zurückbewegt.

Das Gehäuseteil 18 bildet vorzugsweise ein kappenartiges Gehäuseunterteil und um- bzw. übergreift einen unteren freien Endbereich des Behälters 3, mit dem es vorzugsweise zusammen in einer Einführrichtung E an bzw. in das Gehäuseoberteil 16 geschoben und befestigt werden kann. Beim Spannen der Antriebsfeder 7 bewegt sich der Behälter 3 mit seinem Endbereich (weiter) in das Gehäuseteil 18 bzw. zu dessen stirnseitigen Ende hin, wobei eine axial wirkende, im Gehäuseteil 18 angeordnete Feder 20 am Behälterboden 21 zur Anlage kommt und mit einem Anstechelement 22 den Behälter 3 bzw. eine bodenseitige Versiegelung bei der erstmaligen Anlage zur Belüftung ansticht.

Der Zerstäuber 1 weist eine Überwachungseinrichtung 23 auf, die Betätigungen des Zerstäubers 1 zählt, vorzugsweise indem sie ein Drehen des Innenteils 17 gegenüber dem Gehäuseoberteil 16 erfasst. Die Überwachungseinrichtung 23 arbeitet beim Darstellungsbeispiel rein mechanisch.

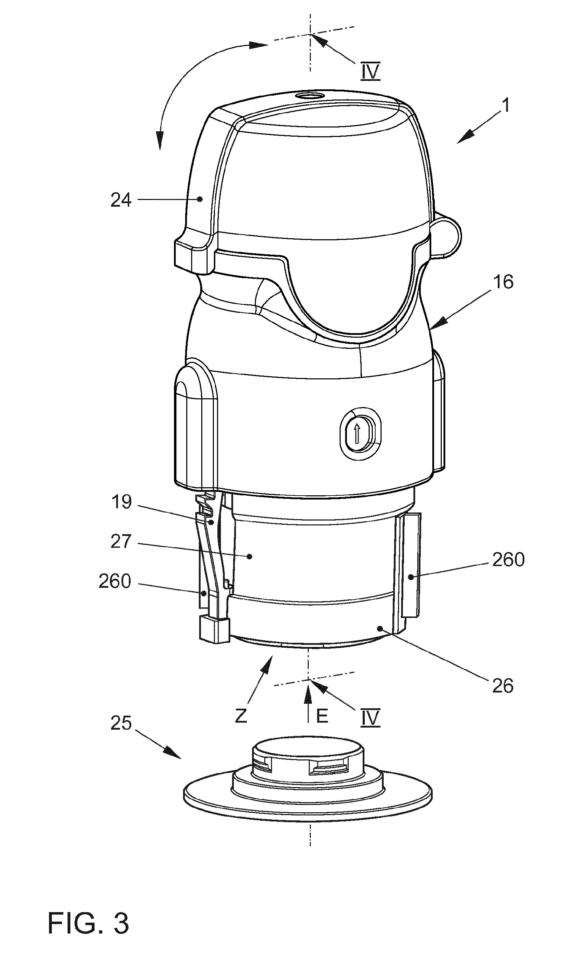

Nachfolgend werden der Aufbau und die Funktionsweise mehrerer Ausführungsformen eines vorschlagsgemäßen Zerstäubers 1 näher erläutert. Dabei wird auf die Fig. 3 bis 43 Bezug genommen. Es werden jedoch nur wesentliche Unterschiede gegenüber dem bekannten Zerstäuber 1 gemäß der Fig. 1 und 2 herausgestellt. Die Ausführungen zu den Fig. 1 und 2 gelten also entsprechend bzw. ergänzend.

Zunächst soll auf die Fig. 3 bis 5 Bezug genommen werden.

Darin ist ein erstes, besonders bevorzugtes Ausführungsbeispiel eines vorschlagsgemäßen Zerstäubers 1 dargestellt.

Abweichend von dem eingangs anhand der Figuren 1 und 2 erläuterten, bekannten Zerstäuber, ist dieses Ausführungsbeispiel der Erfindung (wie auch die darauffolgenden Varianten) für den Einsatz mit wechselnden Einheiten aus Gehäuseunterteil und Wirkstoff-Behälter ausgebildet.

Vom Zerstäuber 1 ist das Oberteil 16 ersichtlich, welches im Bereich des Mundstücks 13 mit einer schwenkbaren Kappe 24 abdeckbar ist.

Im Innern des Gehäuses 16 sind der Druckerzeuger 5 mitsamt dem Förderrohr 9, der Halterung 6 und der Antriebsfeder 7 ersichtlich, wobei ein Verschlussring 17a als unterer Anschlag für die Antriebsfeder 7 dient.

Im Ausführungsbeispiel wird durch den Verschlussring 17a eine Zugangsöffnung Z zum Einsetzen eines Behälters mit Wirkstoff umrahmt. Ferner ist das drehbare Innenteil 17 ersichtlich, welches teilweise unten aus dem Gehäuseoberteil 16 herausragt und durch einen Abdeckring 27 teilweise abgedeckt wird.

An den Abdeckring 27 ist einstückig ein rastend nachgiebiges Halteelement 19 angeformt, welches, wie bereits erwähnt, zur lösbaren Befestigung eines nicht dargestellten Gehäuseunterteils dient.

Stirnseitig am Abdeckring 27 ist ein Kodierring 26 angeordnet, an den zwei radial abstehende, flügelartige Kodierelemente 260 einstückig angeformt sind.

In Fig. 3 ist ein Verschlusselement 25 ersichtlich, welches in einer Einsetzrichtung E von unten stopfenartig in die Zugangsöffnung Z eingesetzt wird und die Zugangsöffnung Z verschließt.

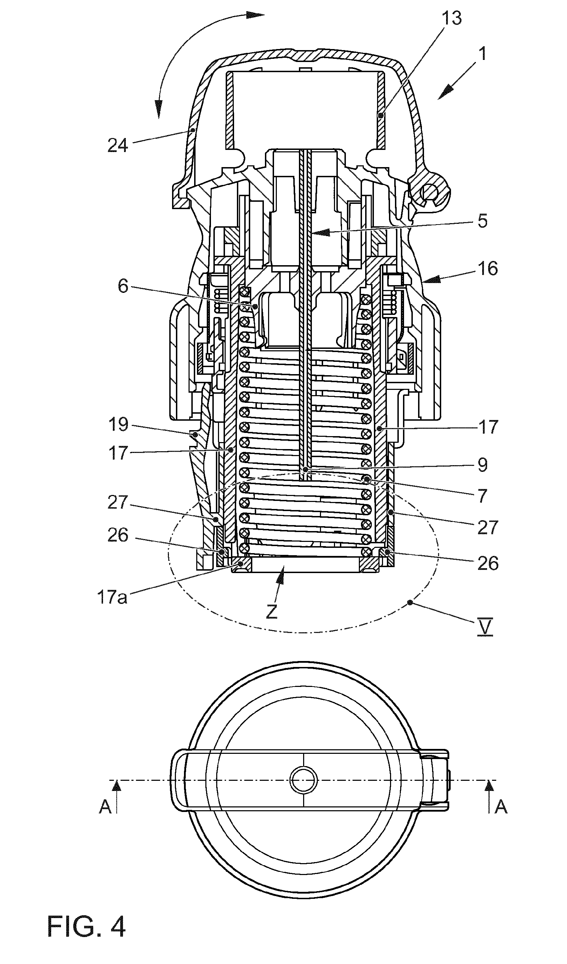

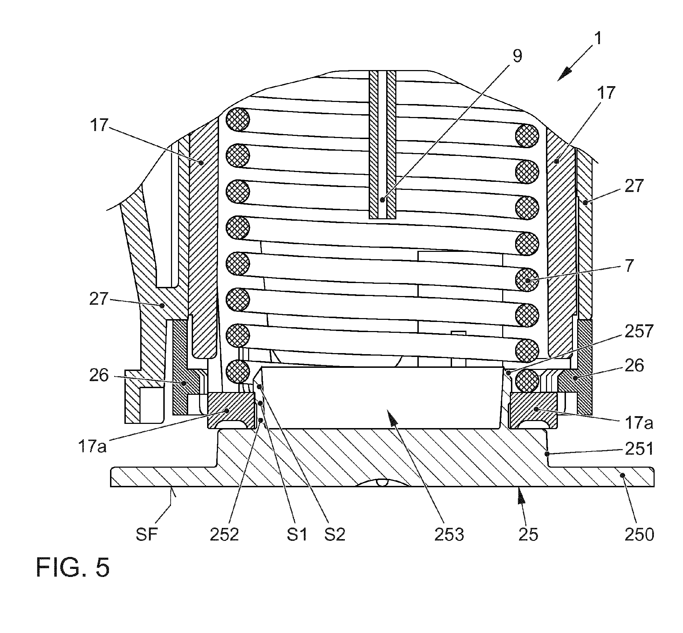

Insbesondere aus den Figuren 4 und 5 ist erkennbbar, dass die Zugangsöffnung Z im Ausführungsbeispiel radial durch den Verschlussring 17a begrenzt wird und das Verschlusselement 25 zu einem Teil in diese Zugangsöffnung Z eingesetzt ist. Dies wird später anhand von Fig. 5 noch näher erläutert.

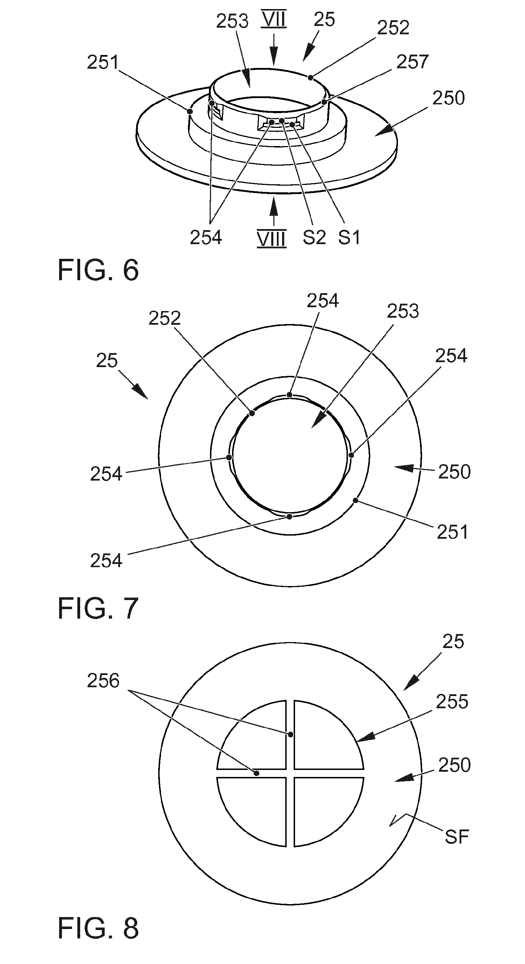

Wie aus den Fig. 6 bis 8 ersichtlich ist, weist das Verschlusselement 25 im Umriss eine kreisrunde Form auf. Dabei ist zunächst ein flacher, kreisringförmiger Grundkörper 250 ausgebildet, an den sich radial nach innen ein umlaufender Absatz 251 anschließt. Noch weiter radial nach innen erhebt sich von diesem Absatz 251 eine kreisringförmige Wandung 252, durch die ein Innenraum 253 gebildet wird.

An die Wandung 252 sind gleichmäßig über ihren Umfang verteilt, radial außenseitig vier Befestigungselemente 254 angeformt. Jedes Befestigungselement 254 weist dabei zwei Stufen S1 und S2 auf. Die Stufen S1 und S2 vergrößern sich radial in Einsetzrichtung E. Mit anderen Worten weist, ausgehend vom umlaufenden Absatz 251, die in Einsetzrichtung E zuerst auftretende Stufe S1 eine geringere radiale Abmessung auf als die in Einsetzrichtung E folgende, zweite Stufe S2.

Abweichend vom Ausführungsbeispiel kann auch eine andere Anzahl Stufen vorgesehen sein.

In Einsetzrichtung E hinter der zweiten Stufe S2 ist eine umlaufende Einführschräge 257 vorhanden.

Auf der Unterseite wird durch den flachen, kreisringförmigen Grundkörper 250 eine plane Standfläche SF ausgebildet. Anzumerken ist, dass der Grundkörper 250 einen deutlich größeren Durchmesser als das Innenteil 17 aufweist. Auf diese Weise wird die Standfläche SF im Vergleich zum durch das Innenteil 17 gebildeten Kreisring als vergleichsweise breiter, großer Ring ausgebildet und eine gute Standsicherheit des Zerstäubers 1 mit montiertem Verschlusselement 25 erzielt.

Dabei ist es allerdings aus verpackungstechnischen Gründen von Vorteil, wenn der Durchmesser des Grundkörpers 250 nicht größer ist als die größten, radialen Abmessungen am Zerstäuber 1, also insbesondere des Gehäuseoberteils 16 und/oder der Kappe 24.

An diese Standfläche SF schließt sich radial nach innen eine kreisförmige Vertiefung 255 an. Die Vertiefung 255 ist von sich rechtwinklig kreuzenden Rippen 256 durchzogen.

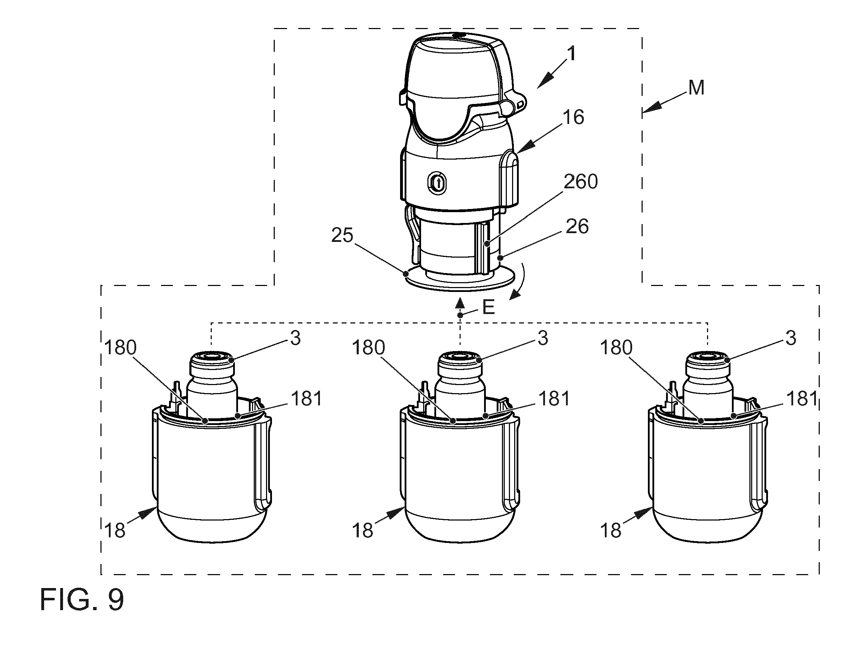

Durch die Rippen 256 wird von vornherein ein eventueller Versuch des Kunden unterbunden, einen Wirkstoff-Behälter 3 auch nur teilweise in die Vertiefung 255 einzusetzen. Spätestens durch die "haptische Rückmeldung" wird klar, dass das Verschlusselement 25 erst entfernt werden muss, bevor ein Behälter 3 in den Zerstäuber 1 eingesetzt werden kann. Überdies wird durch die Rippen 256 für einen Benutzer in aller Regel bereits vorher optisch signalisiert, dass das Verschlusselement 25 nicht zum Durchstoßen, beispielsweise mittels eines Behälters 3 gedacht ist, sondern dass das Verschlusselement 25 vorher zu entfernen ist (vergleiche auch Fig. 9).

Wie aus Fig. 5 ersichtlich, ragt im montierten Zustand das montierte Verschlusselement 25 mit der Wandung 252 in die durch den Verschlussring 17a gebildete Zugangsöffnung Z hinein, wobei sich das Verschlusselement 25 mittels der ersten Stufe S1 eines jeden Befestigungselementes 254 klemmend am Innenradius des Verschlussrings 17a und mittels der zweiten Stufe S2 eines jeden Befestigungselementes 254 rastend an der umlaufenden Oberkante des Verschlussrings 17a festhält. Dabei stützt sich das Verschlusselement 25 in Einsteckrichtung E gleichzeitig mit dem Absatz 251 gegen den Verschlussring 17a ab. Die Einführschräge 257 erleichtert das vorherige Einsetzen des Verschlusselementes 25 in den Verschlussring 17a. Optional kann an der zweiten Stufe S2, quasi als Übergang von der zweiten Stufe S2 zur ersten Stufe S1, noch eine weitere Schräge vorhanden sein, mit der das Herausziehen des Verschlusselementes 25 erleichtert werden kann (nicht dargestellt).

Das Verschlusselement 25 verschließt die Zugangsöffnung Z stopfenartig.

In Fig. 9 ist nunmehr eine Montage- bzw. Verkaufseinheit M dargestellt, welche aus einem erfindungsgemäßen Zerstäuber 1 mit eingesetztem Verschlusselement 25 und drei Gehäuseunterteilen 18 besteht. Abweichend vom Ausführungsbeispiel kann die Montageeinheit M jedoch auch mehr oder weniger Gehäuseteile 18 aufweisen.

Jedes der Gehäuseteile 18 weist einen Behälter 3 mit einem bestimmten, für den Patienten notwendigen Wirkstoff auf. Dabei ist jeder Behälter 3 für den Patienten unlösbar (d. h. nur mit einem Spezialwerkzeug lösbar) mit dem Gehäuseteil 18 verbunden.

Ferner weist jedes Gehäuseteil 18 vorzugsweise ein Kodiemittel auf, das derartig ausgebildet ist, dass das Gehäuseteil 18 mitsamt Behälter 3 nur dann in den Zerstäuber 1 einsetzbar oder mit diesem verwendbar ist, wenn dieser ein eigenes Kodiermittel aufweist, das eine zum Kodiermittel am Gehäuseteil 18 passende Kodierung aufweist. In einer Ausführungsform umfasst dieses Kodiermittel am Gehäuseteil 18 eine Kodierhülse 180, welche mit nutartigen Kodierelementen 181 versehen ist, die mit den Kodierelementen 260 derart zusammenpassen, dass das Gehäuseteil 18 problemlos auf den Zerstäuber 1 bzw. auf das Innenteil 17 hin zum Gehäuseoberteil 16 aufgeschoben werden kann. Alternativ sind solche nutartigen Kodierelemente 181 direkt in der Innenwand des Gehäuseteils 18 abgeformt.

Vor dem Aufschieben von Gehäuseteil 18 auf den Zerstäuber 1 bzw. auf das Innenteil 17 muss jedoch das Verschlusselement 25 durch den Patienten vom Gehäuseoberteil 16 abgezogen und damit die Zugangsöffnung Z zum Einführen des Behälters 3 freigegeben werden.

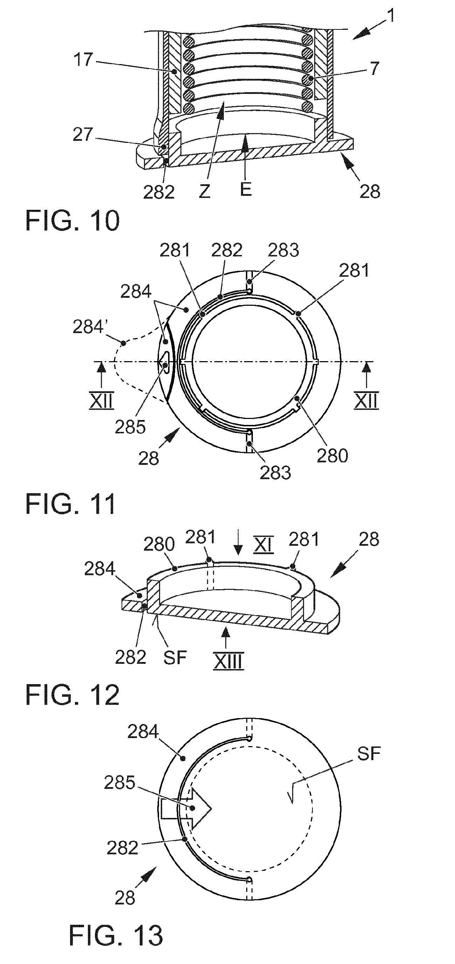

Nunmehr wird auf die Fig. 10 bis 13 Bezug genommen, in der ein weiteres Ausführungsbeispiel der Erfindung dargestellt ist.

So ist ein in einer Draufsicht kreisförmiges Verschlusselement 28 stopfenartig in die Zugangsöffnung Z des Zerstäubers 1 eingesetzt.

Dabei weist das Verschlusselement 28, etwas radial nach innen versetzt eine kreisringförmige Wandung 280 auf, an die radial außenseitig sechs Stege 281, gleichmäßig um den Umfang verteilt, angeformt sind.

Die Stege 281 sind dabei parallel zur Einsetzrichtung E angeordnet.

Im Ausführungsbeispiel klemmen sich die Stege 281 radial innenseitig gegen eine Wandung des Abdeckrings 27.

Auf der Unterseite des Verschlusselementes 28 ist eine plane Standfläche SF ausgebildet. Auf der Standfläche SF ist auch ein pfeilartiges Symbol 285 ersichtlich, welches einem Patienten auf die notwendige Entfernung des Verschlusselementes 28 hinweisen soll.

Ferner ist in die Standfläche SF eine halbkreisförmige, schlitzartige Materialausnehmung 282 eingebracht. Dadurch wird eine halbringartige Greiflasche 284 ausgebildet, die nach unten aus der Standfläche SF herausgeklappt und zum leichteren Entfernen des Verschlusselements 28 dienen soll. Mit Hilfe der Greiflasche 284 können beim Entfernen des Verschlusselements 28 vom Zerstäuber 1 größere Passkräfte zwischen Verschlusselement 28 und Zerstäuber 1 überwunden werden.

Zur Erleichterung des Herausklappens sind zwei Filmscharniere 283 vorhanden.

Abweichend vom Ausführungsbeispiel kann der Griffbereich der Griffflasche 284 noch vergrößert werden (vergleichen 284').

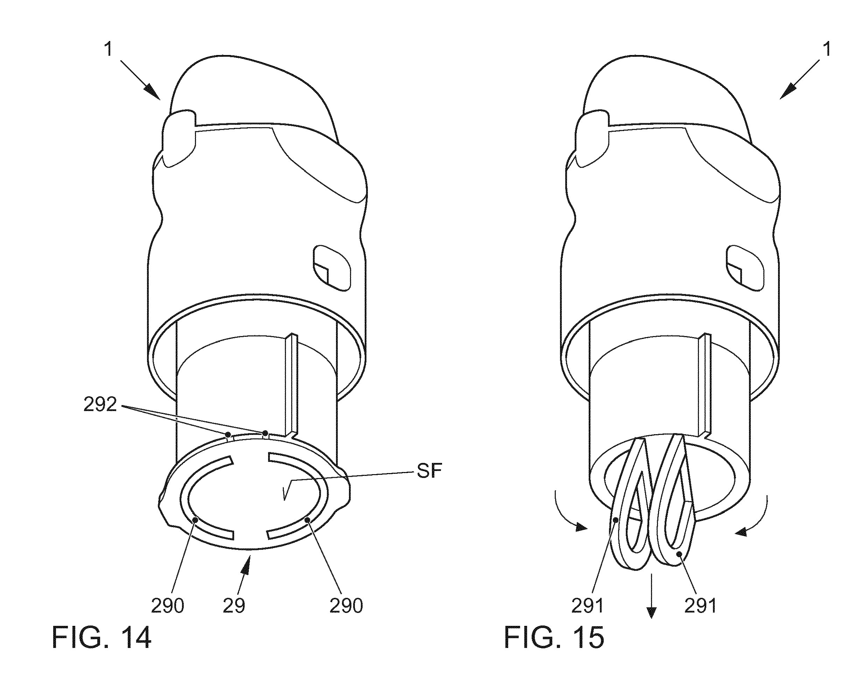

In den Fig. 14 und 15 ist ein Verschlusselement 29 in den Zerstäuber 1 eingesetzt, welches im Unterschied zum vorherigen Beispiel eine Standfläche SF aufweist, welche mit zwei halbkreisförmigen, schlitzartigen Materialausnehmungen 290 versehen ist. Zusätzlich sind an den Seiten Filmscharniere 292 vorhanden. Hierdurch wird es möglich, dass zwei halbringartige Greiflaschen 291 leicht nach unten aus der Standfläche SF herausgeklappt werden können und das Verschlusselement 29 unter optimaler Krafteinleitung nach unten aus dem Zerstäuber 1 herausgezogen werden kann.

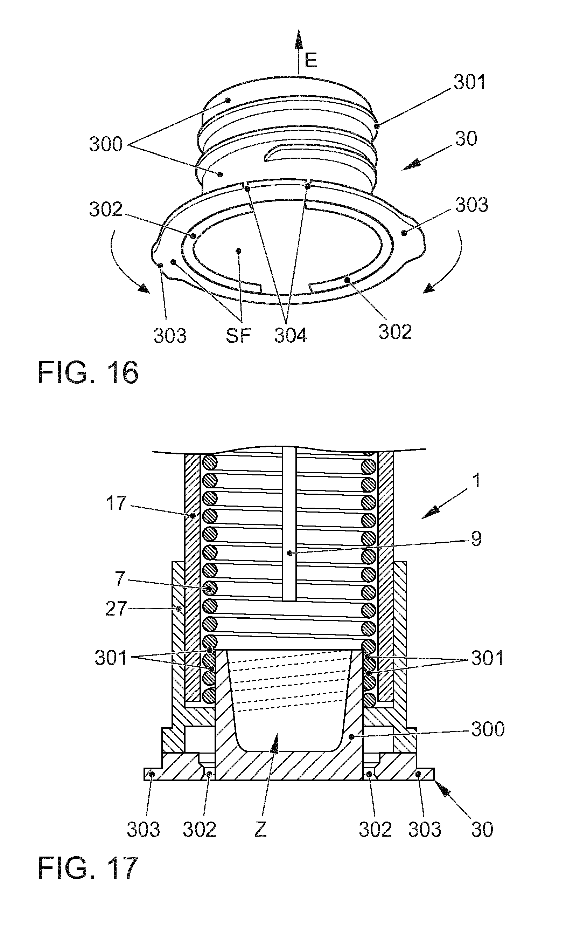

In den Fig. 16 und 17 ist ein weiteres Ausführungsbeispiel der Erfindung dargestellt. Es ist ein Verschlusselement 30 vorhanden, welches in der Draufsicht ebenso eine kreisförmige Gestalt aufweist.

Ausgehend von einer planen Standfläche SF erstreckt sich, etwas radial nach innen versetzt, in Einsetzrichtung E eine kreisringförmige Wandung 300, die mit einer gewindeartigen Anformung 301 versehen ist.

Bevorzugt ist die Breite zwischen den Umläufen der gewindeartigen Anformung 301 so bemessen, dass der Verschlussring 17 a als Ganzes in diesem Gewinde laufen kann, ohne das ein Gegengewinde auf seiner Innenseite abgeformt ist. Auf diese Weise kann das Verschlusselement 30 in den Verschlussring 17a am Zerstäuber 1 hinein gedreht werden.

Ferner ist die Standfläche SF wiederum mit zwei halbkreisförmigen, schlitzartigen Materialausnehmungen 302 versehen, so dass zwei Greifelemente 303, durch Filmscharniere 304 begünstigt, nach unten herausgeklappt werden können und somit ein Entfernen des montierten Verschlusselementes 30 erleichtert wird.

Wie anhand von Fig. 17 dargestellt, hält sich optional das Verschlusselement 30 gewindeartig in den durch die Antriebsfeder 7 gebildeten Zwischenräumen fest. Durch die Greifelemente 303 wird dabei ein unterer Anschlag gegen den Abdeckring 27 gebildet. Ein Lösen und auch ein Einsetzen des Verschlusselementes 30 erfolgt durch entsprechende Verdrehung des Verschlusselementes 30.

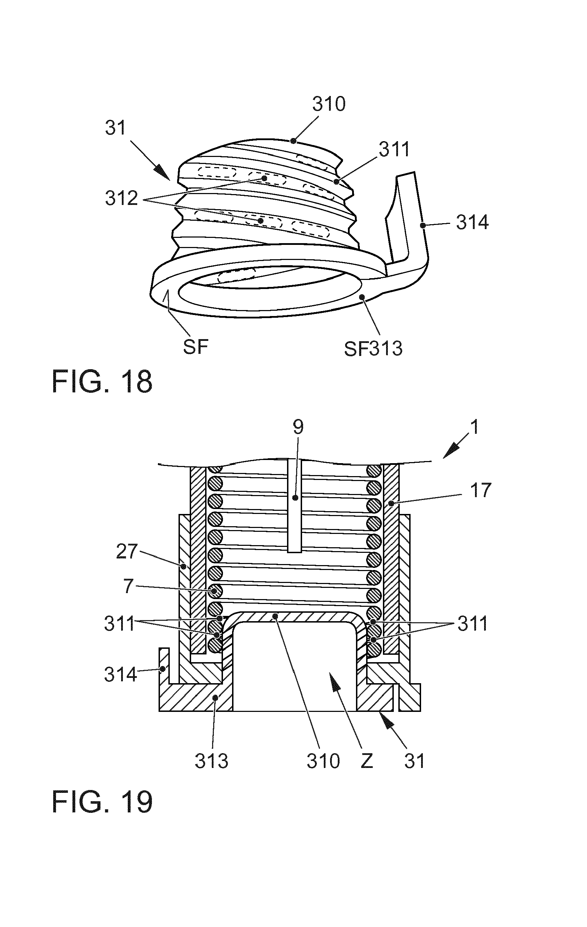

Den Fig. 18 und 19 ist ein weiteres Ausführungsbeispiel entnehmbar, bei dem ein Verschlusselement 31 mit einer spiralförmigen Rastschlinge 311 versehen ist, welche in einem oberen Boden 310 endet.

Zwischen der Rastschlinge 311 sind Sollbruchstellen 312 eingebracht. Auf der dem Boden 310 gegenüberliegenden Seite ist die Rastschlinge 311 mit einem Abschlussring 313 einstückig verbunden, welcher auch eine kreisringförmige, wiederum plane Standfläche SF ausbildet. Der Abschlussring 313 ist wiederum einstückig mit einem Greifelement 314 verbunden.

Das Verschlusselement 31 wird zunächst maschinell in die Zugangsöffnung Z eingepresst.

Zum späteren Entfernen wird das Verschlusselement 31 am Greifelement 314 erfasst und nach unten aus der Zugangsöffnung Z herausgezogen. Dabei brechen die Sollbruchstellen 312 nach Art eines Siegelbruchs auf und der Abschlussring 313 mitsamt spiralförmiger Rastschlinge 311 und Boden 310 kann aus dem Zerstäuber 1 bzw. aus der Zugangsöffnung Z herausgezogen werden.

Wie ersichtlich hält sich das Verschlusselement 31 im montierten Zustand mittels der spiralförmigen Rastschlinge 311 in den spiralförmigen Zwischenräumen der Antriebsfeder 7 fest.

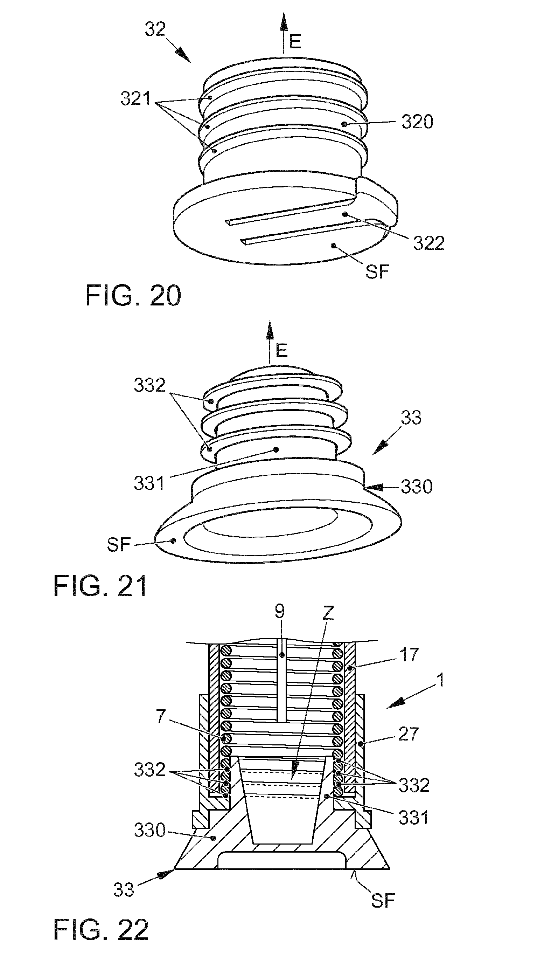

Fig. 20 zeigt ein weiteres Verschlusselement 32, welches stopfenartig in die Zugangsöffnung Z des Zerstäubers 1 einsetzbar ist.

Auch hierbei ist eine im Wesentlichen kreisförmige Standfläche SF vorhanden, von welcher sich in Einsteckrichtung E eine kreisringförmige Wandung 320 erstreckt. An die kreisringförmige Wandung 320 sind lamellenartige Rastelemente 321 angeformt, welche sich im montierten Zustand des Verschlusselementes 32 mit dem spiralförmigen Zwischenraum der Antriebsfeder 7 verrasten.

Ferner ist ein Greifelement 322 Bestandteil der Standfläche SF, welches jedoch nach unten aus der Standfläche SF herausgeklappt werden kann.

In den Fig. 21 und 22 ist eine andere Ausführungsform eines erfindungsgemäßen Zerstäubers 1 mit einem Verschlusselement 33 gezeigt.

Das Verschlusselement 33 weist einen kreisrunden Standfuß 330 mit einer kreisringförmigen Standfläche SF auf. Aus dem Standfuß 330 ragt radial nach innen versetzt eine kreisringförmige Wandung 331 in Einsetzrichtung E hervor, an der wiederum drei umlaufende, lamellenartige Rastelemente 332 angeformt sind.

Im in die Zugangsöffnung Z eingesetzten Zustand verrasten die Rastelemente 332 mit zwischen den Windungen der Antriebsfeder 7 vorhandenen Zwischenräumen und das Verschlusselement 33 stützt sich mit einem umlaufenden Absatz des Standfußes 330 in einer korrespondierenden Ausnehmung des Abdeckrings 27 ab.

Wie ersichtlich, ist der Standfuß 330 im unteren Bereich im Querschnitt ausladend (sich konisch erweiternd) ausgebildet, so dass sich eine im Außendurchmesser vergleichsweise breite Standfläche SF ergibt. Dies führt zu einer guten Standsicherheit.

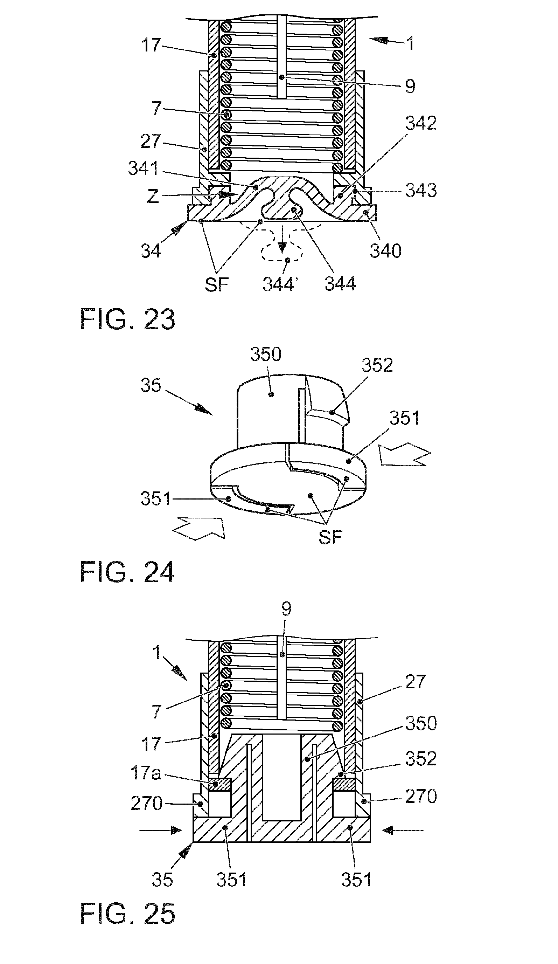

In der Fig. 23 ist eine Ausführungsform der Erfindung dargestellt, bei der die Zugangsöffnung Z des Zerstäubers 1 durch ein Verschlusselement 34 verschlossen ist, welches zwei stabile Stellungen einnehmen kann, also bistabil ausgebildet ist. Vorzugsweise besteht das Verschlusselement 34 aus einem weichen bzw. elastischen Kunststoff.

So weist das Verschlusselement 34 einen kreisringförmigen Boden 340 auf, an den eine kreisringförmige Wandung 342 angeformt ist, welches bevorzugt eine radial nach außen gerichtete, umlaufende Rastkante 343 aufweist.

Mit der Rastkante 343 greift das Verschlusselement 34 vorzugsweise in eine entsprechende, umlaufende Ausnehmung des Abdeckrings 27 rastend ein. Ein Eingriff an entsprechend geformten Ausnehmungen an einem verlängerten Innenteil 17 oder eine Passung mit dem Verschlussring 17a sind alternativ ebenfalls möglich.

Radial innerhalb der kreisringförmigen Wandung 342 ist ein bistabiler Mittelbereich 341 ausgebildet, welcher in der in der Darstellung gezeigten Montagestellung des Verschlusselementes 34 in das Innere des Zerstäubers 1 hineingestülpt ist und an den auswärts ein noppenartiges Greifelement 344 angeformt ist.

Der kreisringförmige Boden 340 bildet zusammen mit der Oberfläche des Greifelementes 344 eine fluchtende Standfläche SF aus.

Zum Entfernen kann das Verschlusselement 34 am Greifelement 344 nach außen in eine zweite stabile Position (344') gezogen werden. Dadurch wird die in den Zerstäuber 1 eingreifende Wandung 342 bzw. die Rastkante 343 nach innen gebogen und die Presspassung bzw. der Eingriff zwischen Verschlusselement 28 und Zerstäuber 1 wird gelöst. Das Verschlusselement 28kann dann aus dem Zerstäuber 1 entfernt werden. Die eingreifende Wandung 342 bildet hierbei gewissermaßen ein Spreizelement, das in der Montage-Stellung des Verschlusselements 34 in aufgespreiztem Zustand vorliegt.

Anhand der Fig. 24 und 25 soll eine weitere Ausführungsform der Erfindung beschrieben werden, bei der ein Verschlusselement 35 die Zugangsöffnung Z des Zerstäubers 1 verschließt.

Das Verschlusselement 35 weist eine im Wesentlichen kreisrunde Standfläche SF auf, wobei die Standfläche SF außenseitig auch durch zwei Bewegungsarme 351 gebildet wird, welche einstückig, jedoch über entsprechende Materialunterbrechungen bewegbar, mit einem Hohlkörper 350 verbunden sind.

Im Verbindungsbereich der Bewegungsarme 351 mit dem Hohlkörper 350 weisen die Bewegungsarme 351 Rasthaken 352 auf.

Zum Einsetzen braucht das Verschlusselement 35 lediglich in die Zugangsöffnung Z eingedrückt zu werden, wobei bedingt durch die zwischen den Bewegungsarmen 351 und dem Hohlkörper 350 angeordneten Materialunterbrechungen, die Bewegungsarme 351 und mit ihnen die Rasthaken 352 radial nach innen nachgeben und nach Überwindung der durch den Verschlussring 17a gebildeten Zugangsöffnung Z wieder rastend radial nach außen ausfahren können.

Im montierten Zustand stützt sich ein Teil der Bewegungsarme 351 stirnseitig gegen radial nach außen stehende Absätze 270 des Abdeckrings 27 ab.

Zum Lösen des Verschlusselementes 35 ist lediglich ein radiales Zusammendrücken der Bewegungsarme 351 und ein anschließendes Herausziehen des Verschlusselementes 35 notwendig.

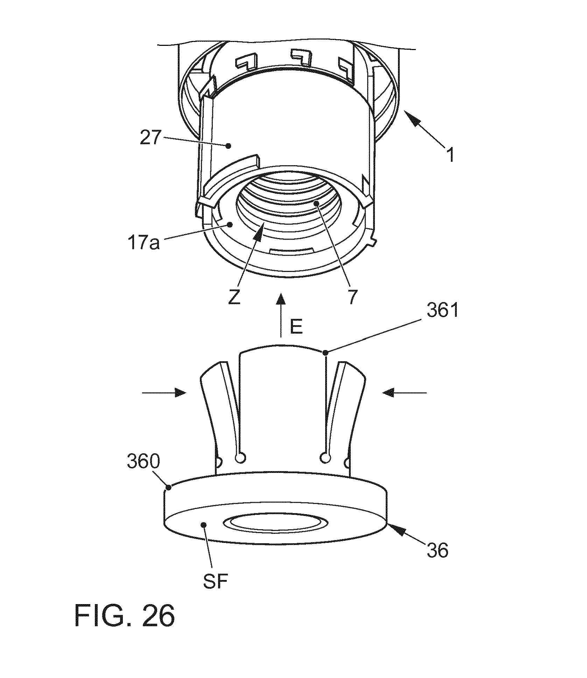

Bei dem in Fig. 26 dargestellten Ausführungsbeispiel wird ein Verschlusselement 36 in die durch den Verschlussring 17a gebildete Zugangsöffnung Z gesteckt.

Das Verschlusselement 36 weist einen kreisförmigen Grundkörper 360 auf, welcher mit einer kreisringförmigen Standfläche SF versehen ist.

Ausgehend von dem Grundkörper 360, radial nach innen versetzt, erstrecken sich mehrere, vorzugsweise vier Biegelaschen 361 in Einsteckrichtung E.

Die Biegelaschen 361 sind an ihrem oberen Ende leicht radial nach außen gebogen und müssen zum Einsetzen in die Zugangsöffnung Z des Zerstäubers 1 etwas zusammengedrückt werden.

Nachdem die Biegelaschen 361 durch die Zugangsöffnung Z hindurchgetreten sind, spreizen sich diese wieder auf und liegen klemmend und/oder rastend an der Antriebsfeder 7 an.

Im montierten Zustand wird der untere Bereich des Abdeckrings 27 vom Grundkörper 360 radial übergriffen.

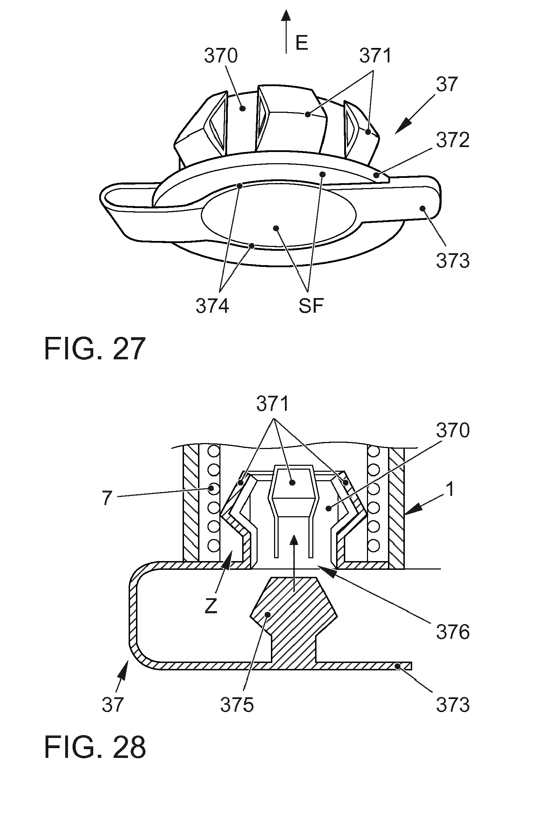

Die Fig. 27 und 28 zeigen eine alternative Ausbildung, bei der ein Verschlusselement 37 in die Zugangsöffnung Z eingesetzt ist.

Das Verschlusselement 37 weist, in Einsteckrichtung E von einem kreisringförmigen Standelement 372 ausgehend, eine Hohlwandung 370 auf, welche über ihren Umfang verteilt mit mehreren elastischen Spreizelementen 371 versehen ist.

Zur Montage wird die Hohlwandung 370 mit den Spreizelementen 371 zunächst in die Zugangsöffnung Z gesteckt und hält sich dort zunächst lose an der Antriebsfeder 7 fest.

Anschließend wird ein Spreizdorn 375 durch eine Öffnung 376 des Verschlusselementes 37 hindurch gesteckt, welcher, bedingt durch seine Formgebung, die Spreizelemente 371 weiter radial nach außen spreizt. Federeigenschaften der Spreizelemente 371 werden im eingesteckten Zustand des Spreizdorns 375 gehemmt, da er, dadurch dass er innen an den Spreizelementen 371 anliegt, über seine Formgebung die radiale Position der Spreizelemente 371 fixiert. Somit führt der Spreizdorn 375 im eingesteckten Zustand zu einer festen Verklemmung der Spreizelemente 371 mit der Antriebsfeder 7.

Der Spreizdorn 375 ist einstückig mit einer Grifflasche 373 verbunden, welche wiederum einstückig mit dem kreisringförmigen Standelement 372 verbunden ist.

Wie ersichtlich werden durch das Standelement 372 und durch die Grifflasche 373 eine plane Standfläche SF ausgebildet.

Die Standfläche SF wird lediglich durch Materialunterbrechungen 374 unterbrochen, welche durch die aus der Standfläche SF herausziehbare Grifflasche 373 bedingt sind.

Nach Herausziehen der Grifflasche 373 und mithin des Spreizdorns 375 aus der Hohlwandung 370, wird die beschriebene Spreizwirkung wieder aufgehoben und das Verschlusselement 37 kann mit Hilfe der Grifflasche 373 wieder aus der Zugangsöffnung Z herausgezogen werden.

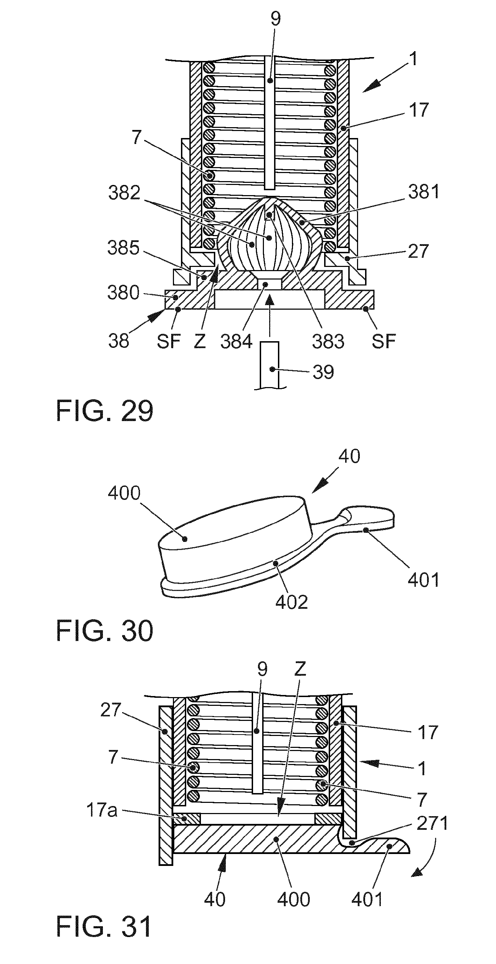

Die Fig. 29 zeigt ein in den Zerstäuber 1 eingesetztes Verschlusselement 38, welches mit einem kreisringartigen Standelement 380 ausgestattet ist. Durch das Standelement 380 wird eine kreisringförmige Standfläche SF ausgebildet.

An das Standelement 380 schließt sich, radial nach innen versetzt, ein umlaufender Absatz 385 an, welcher mittig mit einer Durchgangsöffnung 384 versehen ist.

Wiederum radial nach innen versetzt ist an den Absatz 385 ein Rastteil 381 angeformt, welches eine Vielzahl von nach außen gebogenen, in Einsetzrichtung des Verschlusselementes 38 weisende Streifenelemente 382 aufweist.

Die Streifenelemente 382 sind oben zu einem Dornansatz 383 zusammengeführt, welcher mit der Durchgangsöffnung 384 radial in etwa fluchtet.

In der gezeigten Montageposition klemmen sich die nach außen gebogenen Streifenelemente 382 elastisch gegen die Antriebsfeder 7 und das Standelement 380 bzw. der umlaufende Absatz 385 werden stirnseitig gegen den Abdeckring 27 gedrückt. Gewissermaßen bilden die Streifenelemente Spreizelemente, die in der Montageposition aufgespreizt vorliegen.

Zur Montage/Demontage ist es erforderlich, dass ein Montagedorn 39 durch die Öffnung 384 hindurchgesteckt und axial gegen den Dornansatz 383 gedrückt wird. Hierdurch wird das Rastteil 381 gelängt (in die Länge gezogen) und dadurch in seinem Außendurchmesser verringert.

Im derart gelängten Zustand kann das Rastteil 381 leicht durch die Zugangsöffnung Z hindurchgesteckt bzw. aus dieser wieder herausgezogen werden.

Aus den Fig. 30 und 31 ist eine Ausführungsform ersichtlich, bei der ein Verschlusselement 40 die Zugangsöffnung Z des Zerstäubers 1 abdeckt.

Das Verschlusselement 40 weist einen stopfenartigen Grundkörper 400 auf, welcher unter Bildung eines umlaufenden Absatzes 402 einstückig mit einem laschenartigen Greifelement 401 verbunden ist.

Der Grundkörper 400, welcher kreisförmig ausgebildet ist, ist in seinem Außendurchmesser geringfügig größer als ein entsprechender Innendurchmesser des Abdeckrings 27 und wird in diesen eingepresst, wobei er stirnseitig gegen den Verschlussring 17a anliegt. In dem Abdeckring 27 ist lediglich an einer Stelle eine Ausnehmung 271 vorgenommen, durch die das Greifelement 401 radial nach außen ragen kann.

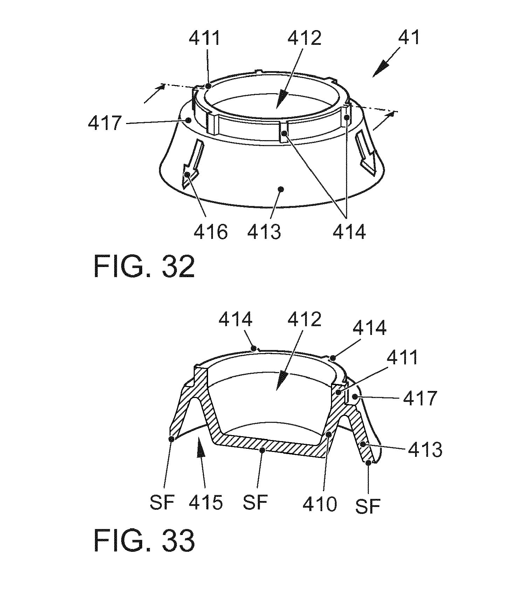

Die Fig. 32 und 33 zeigen ein Verschlusselement 41, welches ebenso in einem Zerstäuber 1 (nicht dargestellt), die Zugangsöffnung Z verschließend, eingesetzt werden kann.

Das Verschlusselement 41 weist einen napfartigen Grundkörper 410 auf mit einem Innenraum 412, an den radial außenseitig eine radial nach außen geneigte, umlaufende Seitenwandung 413 angeformt ist, so dass ein v-förmiger Zwischenraum 415 entsteht.

An den Grundkörper 410 ist eine kreisringförmige Wandung 411 angeformt, welche von der Seitenwandung 413 radial nach innen versetzt ist, so dass sich ein umlaufender Absatz 417 ausbildet.

An der Wandung 411 sind radial außenseitig parallel zur Einsetzrichtung E ausgerichtete Klemmrippen 414 (vorzugsweise sechs) gleichmäßig über den Umfang verteilt angeordnet, mit denen sich das Verschlusselement 41 klemmend an einer Innenwandung des Zerstäubers 1 im Bereich der Zugangsöffnung Z festhalten kann. Dabei dient der umlaufende Absatz 417 als stirnseitiger Anschlag.

Die Seitenwandung 413 ist ferner mit pfeilartigen Symbolen 416 versehen, welche auf ein notwendiges Entfernen des Verschlusselementes 41 vor dem bestimmungsgemäßen Gebrauch des Zerstäubers 1, das heißt vor Einsetzen eines Behälters 3 mit einem bestimmten Wirkstoff, hinweisen sollen.

Der Boden des napfartigen Grundkörpers 410 und der Boden der umlaufenden Seitenwandung 413 fluchten miteinander, so dass dadurch eine plane Standfläche SF ausgebildet wird.

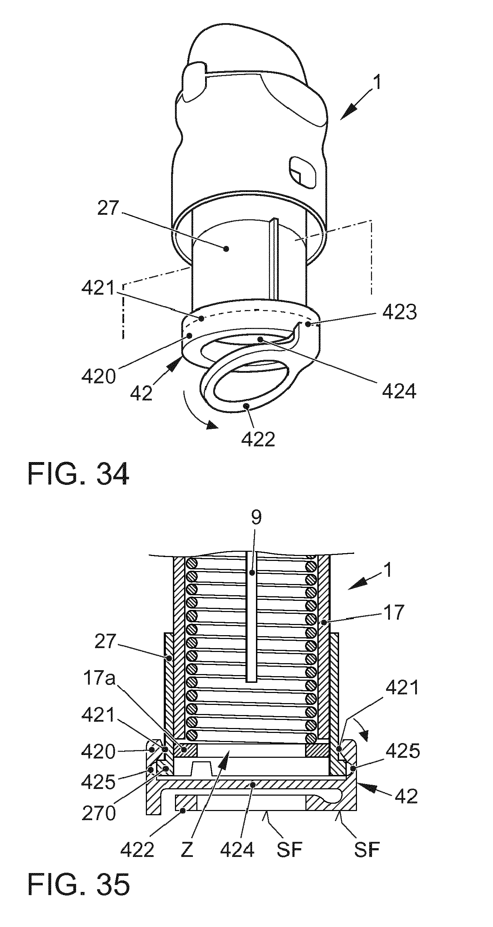

Die Fig. 34 und 35 zeigen ein Ausführungsbeispiel, bei dem die Zugangsöffnung Z des Zerstäubers 1 durch ein Verschlusselement 42 verschlossen ist, welches kappenartig auf den Zerstäuber 1 aufgesetzt ist. Insbesondere ist das Verschlusselement 42 dabei so auf den Abdeckring 27 bzw. so auf das Innenteil 17 aufgesetzt, dass das Verschlusselement 42 den Abdeckring 27 bzw. das Innenteil 17 an der Verbindungsstelle bzw. im Bereich der Zugangsöffnung Z radial von außen umschließt.

Konkret weist das Verschlusselement 42 eine kreisringförmige Wandung 420 auf, wobei in etwa in der Mitte ihrer Länge ein Boden 424 angeordnet ist.

Am oberen Ende der Wandung 420 ist eine radial nach innen gerichtete, umlaufende Rastkante 421 angeordnet, mit der das Verschlusselement 42 einen radial nach außen stehenden Absatz 270 des Abdeckrings 27 radial von außen rastend hintergreift.

Zusätzlich ist an der Wandung 420 innenseitig eine umlaufende Materialschwächung 425 eingebracht, welche ein radiales Auseinanderbewegen der Wandung 420 und damit der Rastkante 421 erleichtern soll.

Zur Montage des Verschlusselementes 42 ist ein einfaches Aufstecken auf den Abdeckring 27 ausreichend.

Zur Demontage des Verschlusselementes 42 ist ein über ein Filmscharnier 423 herausklappbares, ringförmiges Greifelement 422 vorhanden, mit dessen Hilfe das Verschlusselement 42 vom Zerstäuber 1 abgezogen werden kann.

Im Montagezustand wird durch die Wandung 420 und das in diese eingelassene Greifelement 422 ebenfalls eine stabile Standfläche SF ausgebildet.

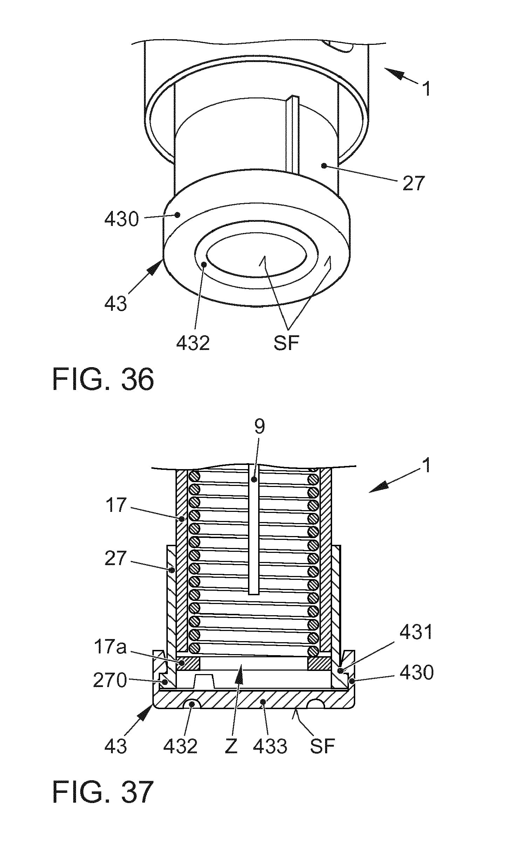

Aus den Fig. 36 und 37 ist ein Ausführungsbeispiel eines erfindungsgemäßen Zerstäubers 1 mit einem Verschlusselement 43 ersichtlich, welches ebenfalls kappenartig auf den Abdeckring 27 aufgesetzt ist und dabei die Zugangsöffnung Z verschließt.

Das Verschlusselement 43 weist einen kreisförmigen Boden 433 auf, an den randseitig eine kreisringförmige Wandung 430 angeformt ist. Am oberen Ende der Wandung 430 ist eine radial nach innen gerichtete, umlaufende Rastkante 431 eingebracht, welche sich im Montagezustand des Verschlusselementes 43 rastend hinter einem umlaufenden Absatz 270 des Abdeckrings 27 festhält.

Der Boden 433 weist eine kreisringförmige Materialschwächung 432 auf, welche eine Standfläche SF unterbricht, jedoch zum rastenden, radialen Nachgeben der Wandung 430 beiträgt.

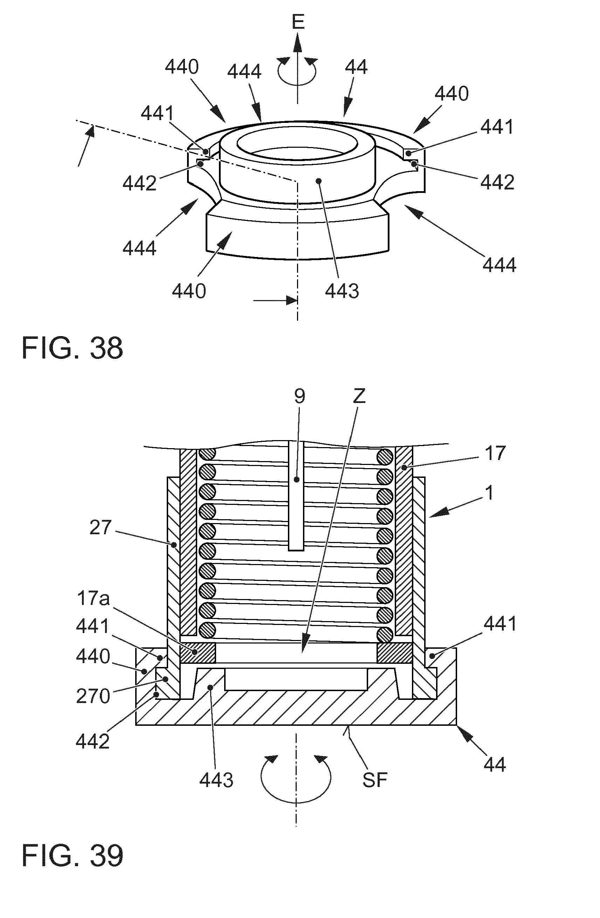

Gemäß den Fig. 38 und 39 ist die Zugangsöffnung Z des Zerstäubers 1 mittels eines Verschlusselementes 44 bajonettartig verschlossen.

So weist das Verschlusselement 44, ausgehend von einer kreisringförmigen Wandung 443 drei radial abstehende Verriegelungsbereiche 440 und entsprechend drei dazwischen liegende Freigabebereiche 444 auf.

Jeder Verriegelungsbereich 440 weist einen radial nach innen stehenden Absatz 441 auf, wodurch eine Radialnut 442 ausgebildet wird.

Entsprechend ist der Abdeckring 27 des Zerstäubers 1 mit einem bereichsweise unterbrochenen radial nach außen abstehenden Absatz 270 ausgebildet (in dieser Darstellung sind die Unterbrechungen nicht ersichtlich), derart, dass an seinen Unterbrechungen die Verriegelungsbereiche 440 in Einsetzrichtung E eingeführt und durch anschließendes Verdrehen des Verschlusselementes 44 die radial nach außen stehenden Absätze 270 in Eingriff mit den Radialnuten 442 gebracht werden können.

Eine Demontage des Verschlusselementes 44 erfolgt in entgegengesetzter Reihenfolge.

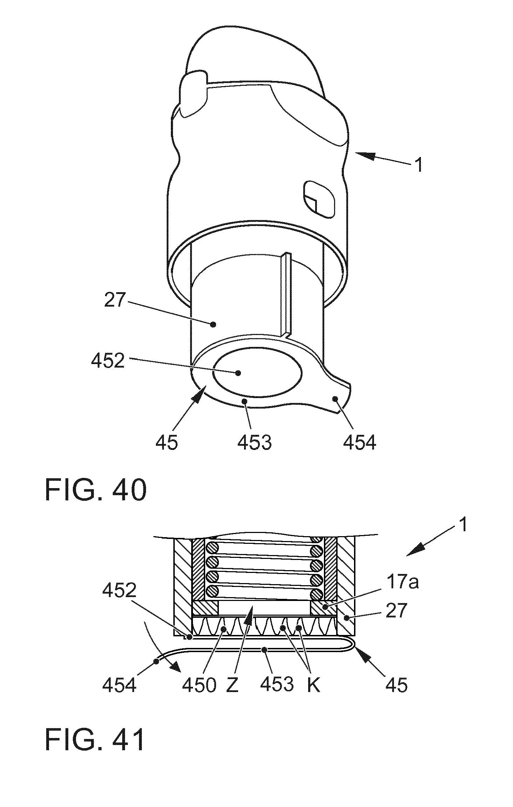

Bei dem in den Fig. 40 und 41 gezeigten Ausführungsbeispiel ist die Zugangsöffnung Z des Zerstäubers 1 durch ein Verschlusselement 45 verschlossen, welches stopfenartig in den Zerstäuber 1 eingedrückt und mit diesem verklebt ist.

Konkret ist das Verschlusselement 45 als Zweikomponententeil ausgebildet, wobei ein kreisrundes Pappelement 450 mit einer radial außenseitig aufgebrachten Klebstoffschicht K versehen ist, wobei das Pappelement 450 fest mit einer radial über das Pappelement 450 überstehenden Kunststofffolie 452 verbunden ist, welche wiederum einstückig in eine kreisringförmige Grifflasche 453 übergeht, an die ein überstehender Griffbereich 454 angeformt ist.

Durch die dünne Ausbildung der Grifflasche 453 hindert diese den Zerstäuber 1 einerseits nicht an einem sicheren Stand, andererseits ist diese ausreichend stabil, um ein Herausziehen des Verschlusselementes 45 aus dem Zerstäuber 1 zu gewährleisten.

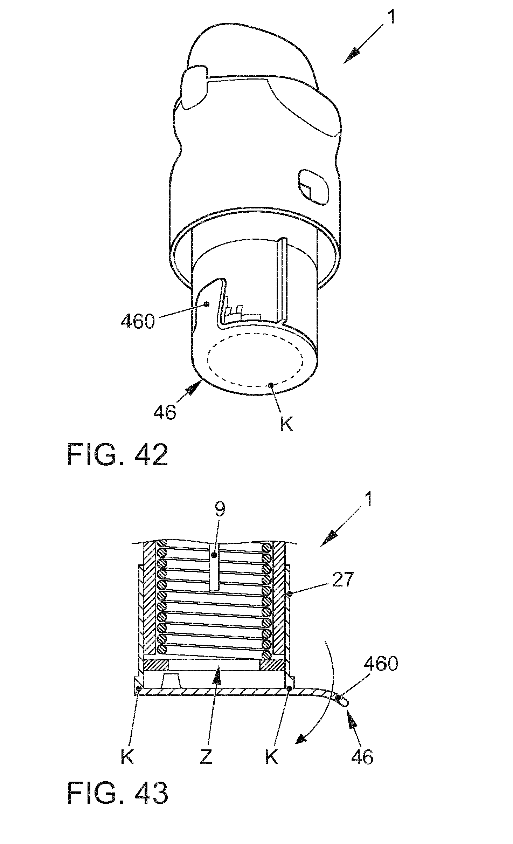

Als letztes Ausführungsbeispiel ist in den Fig. 42 und 43 ein erfindungsgemäßer Zerstäuber 1 dargestellt, dessen Zugangsöffnung Z mit einem als Siegelfolie ausgebildeten Verschlusselement 46 verschlossen ist. Die Siegelfolie 46 weist ebenfalls eine Klebstoffschicht K auf, mittels derer sie stirnseitig mit dem Abdeckring 27 verklebt ist. Zum Entfernen der Siegelfolie 46 ist einstückig eine Grifflasche 460 angeformt.

Sämtlichen Ausführungsbeispielen ist entnehmbar, dass das die Zugangsöffnung Z jeweils verschließende Verschlusselement im Wesentlichen kreisförmig ausgebildet ist, was zu einer kompakten Bauform und zu einem sicheren Stand des Zerstäubers 1 beiträgt. Jedoch sind abweichend davon auch andere Formen durchaus denkbar.

Die Erfindung ist nicht auf das bzw. die obigen Ausführungsbeispiele beschränkt. Diese wurden nur zur allgemeinen Erläuterung des Kerngedankens der Erfindung herangezogen. Die Erfindung kann im Rahmen ihres Schutzumfangs vielmehr auch andere als die zuvor beschriebenen Ausführungsbeispiele bzw. Ausprägungen annehmen. Hierbei kann sie insbesondere auch solche Merkmale aufweisen, die eine Kombination aus Einzelmerkmalen der jeweiligen Ansprüche darstellen.

Bevorzugt ist das mit dem Zerstäuber 1 zu zerstäubende Fluid 2 eine flüssige medizinische Formulierung, die mindestens ein Bestandteil aufweist, das in der Offenbarung der WO 2009/115200 A1, insbesondere auf den Seiten 25 bis 40, oder der EP 2 614 848 A1 in den Absätzen 0040 bis 0087 genannt wird, oder die einer der dort genannten Formulierungen entspricht. Der Inhalt jener Zeilen wird hiermit vollumfänglich, auch zur Aufnahme von Merkmalen, in die vorliegende Anmeldung mit aufgenommen. Insbesondere, können die Formulierungen wässrige oder nicht-wässrige Lösungen oder Mischungen sein, Ethanol enthalten oder frei von Lösemitteln sein.

**Bezugszeichenliste**

| | | | |
|---|---|---|---|
| 1 | Zerstäuber | 13 | Mundstück |
| 2 | Fluid | 14 | Aerosol |
| 3 | Behälter | 15 | Zuluftöffnung |
| 4 | Beutel | 16 | Gehäuseoberteil |
| 5 | Druckerzeuger | 17 | Innenteil |
| 6 | Halterung | 17a | Verschlussring als unterer Anschlag für die Antriebsfeder |
| 7 | Antriebsfeder | | |
| 8 | Sperrelement | 18 | lösbares Gehäuseteil (Unterteil) |
| 9 | Förderrohr | 180 | Kodierhülse |
| 10 | Rückschlagventil | 181 | nutartige Kodierelemente |
| 11 | Druckkammer | 19 | Halteelement |
| 12 | Austragsdüse | 20 | Feder (im Gehäuseunterteil) |
| 21 | Behälterboden | 271 | Ausnehmung |
| 22 | Anstechelement | 28 | Verschlusselement |
| 23 | Überwachungseinrichtung | 280 | kreisringförmige Wandung |
| 24 | Kappe | 281 | angeformte Stege |
| 25 | Verschlusselement | 282 | halbkreisartige Materialausnehmung |
| 250 | flacher, kreisringförmiger Grundkörper | | |
| | | 283 | Filmscharniere |
| 251 | umlaufender Absatz | 284 | Grifflasche |
| 252 | kreisringförmige Wandung | 285 | Symbol |
| 253 | Innenraum | 29 | Verschlusselement |
| 254 | Befestigungselemente | 290 | halbkreisartige Materialausnehmungen |
| 255 | kreisförmige Vertiefung | | |
| 256 | Rippen | 291 | Grifflaschen |
| 257 | Einführschräge | 292 | Filmscharniere |
| 26 | Kodierring | | |
| 260 | Kodierelemente | | |
| 27 | Abdeckring | | |
| 270 | radial nach außen stehender Absatz | | |
| 30 | Verschlusselement | 344 | Greifelement |
| 300 | kreisringförmige Wandung | 35 | Verschlusselement |
| 301 | gewindeartige Anformung | 350 | Hohlkörper |
| 302 | halbkreisartige Materialausnehmung | 351 | Bewegungsarme |
| | | 352 | Rasthaken |
| 303 | Greifelemente | 36 | Verschlusselement |
| 304 | Filmscharniere | 360 | kreisförmiger Grundkörper |
| 31 | Verschlusselement | 361 | Biegelaschen |
| 310 | Boden | 37 | Verschlusselement |
| 311 | spiralförmige Rastschlinge | 370 | Hohlwandung |
| 312 | Sollbruchstellen | 371 | Spreizelemente |
| 313 | Abschlussring | 372 | Standelement |
| 314 | Greifelement | 373 | Grifflasche |
| 32 | Verschlusselement | 374 | Materialunterbrechung |
| 320 | kreisringförmige Wandung | 375 | Spreizdorn |
| 321 | lamellenartige Rastelemente | 376 | Öffnung |
| 322 | Greifelement | 38 | Verschlusselement |
| 33 | Verschlusselement | 380 | Standelement |
| 330 | Standfuß | 381 | Rastteil |
| 331 | kreisringförmige Wandung | 382 | vertikale, nach außen gebogene Streifenelemente |
| 332 | lamellenartige Rastelemente | | |
| 34 | Verschlusselement | 383 | Dornansatz |
| 340 | kreisringförmiger Boden | 384 | Öffnung |
| 341 | bistabiler Mittelbereich | 385 | umlaufender Absatz |
| 342 | kreisringförmige Wandung | 39 | Montagedorn |
| 343 | umlaufende Rastkante | | |
| 40 | Verschlusselement | 440 | Verriegelungsbereiche |
| 400 | stopfenartiger Grundkörper | 441 | radial nach innen stehende Absätze |
| 401 | Greifelement | | |
| 402 | umlaufender Absatz | 442 | Radialnut |
| 41 | Verschlusselement | 443 | kreisringförmige Wandung |
| 410 | napfartiger Grundkörper | 444 | Freigabebereiche |
| 411 | kreisringförmige Wandung | 45 | Verschlusselement |
| 412 | Innenraum | 450 | Pappelement |
| 413 | umlaufende Seitenwandung | 452 | Kunststofffolie |
| 414 | Klemmrippen | 453 | kreisringartige Grifflasche |
| 415 | v-förmiger Zwischenraum | 454 | überstehender Griffbereich |
| 416 | Symbol | 46 | Verschlusselement |
| 417 | umlaufender Absatz | 460 | Grifflasche |
| 42 | Verschlusselement | | |
| 420 | kreisringförmige Wandung | E | Einführ- bzw. Einsetzrichtung des Behälters oder des Verschlusselementes in das Gehäuseoberteil bzw. Innenteil |
| 421 | umlaufende Rastkante | | |
| 422 | ringförmiges Greifelement | | |
| 423 | Filmscharnier | | |
| 424 | Boden | K | Klebstoff |
| 425 | umlaufende Materialschwächung | M | Montage- und Verkaufseinheit |
| 43 | Verschlusselement | SF | Standfläche |
| 430 | kreisringförmige Wandung | S1 | erste Stufe |
| 431 | Rastkante | S2 | zweite Stufe |
| 432 | kreisringförmige Materialschwächung | Z | Zugangsöffnung zum Einsetzen des Behälters oder des Verschlusselementes in das Gehäuseoberteil bzw. Innenteil |
| 433 | Boden | | |
| 44 | Verschlusselement | | |

## Patentansprüche

1. Zerstäuber (1) für ein Fluid (2), in den ein Behälter (3) mit dem Fluid (2) einsetzbar ist, mit einem Druckerzeuger (5) zur Förderung und/oder Zerstäubung des Fluids (2), mit einer Zugangsöffnung (Z) zum Einsetzen des Behälters (3) und mit Befestigungsmitteln (19) zur lösbaren Befestigung eines Gehäuseunterteils (18), **dadurch gekennzeichnet, dass** die Zugangsöffnung (Z) durch ein lösbares Verschlusselement (25,28-38,40-45) verschlossen ist.

2. Zerstäuber (1) nach Anspruch 1, **dadurch gekennzeichnet, dass** sich das Verschlusselement (25,28-38,40-43) klemmend und/oder rastend und/oder gewindeartig am Zerstäuber (1), vorzugsweise im Innern (7,17a, 27) des Zerstäubers, festhält.

3. Zerstäuber (1) nach einem der vorhergehenden Ansprüche, **dadurch gekennzeichnet, dass** sich das Verschlusselement (25,35) klemmend und/oder rastend und/oder gewindeartig an einem Verschlussring (17a) festhält, welcher als unterer Anschlag für eine zum Druckerzeuger (5) gehörige Antriebsfeder (7) dient.

4. Zerstäuber nach einem der vorhergehenden Ansprüche 1 bis 2, **dadurch gekennzeichnet, dass** sich das Verschlusselement (30- 33, 36, 37) rastend, klemmend oder gewindeartig an einer zum Druckerzeuger (5) gehörigen Antriebsfeder (7) festhält.

5. Zerstäuber (1) nach einem der vorhergehenden Ansprüche, **dadurch gekennzeichnet, dass** das Verschlusselement (25,28,41) eine kreisringförmige Wandung (252,280,411) aufweist, an der radial außenseitig wenigstens zwei Befestigungselemente (254,281,414) angeordnet sind, welche sich klemmend und/oder rastend im Bereich der Zugangsöffnung (Z) festhalten.

6. Zerstäuber (1) nach Anspruch 5, **dadurch gekennzeichnet, dass** die Befestigungselemente (254) als an die kreisringförmige Wandung (252) angeformte, stufenartige Elemente ausgebildet sind, mit wenigstens zwei in Einsteckrichtung (E) des Verschlusselementes (25) radial nach außen ansteigenden Stufen (S1, S2).

7. Zerstäuber (1) nach Anspruch 5, **dadurch gekennzeichnet, dass** die Befestigungselemente (281,414) als an die kreisringförmige Wandung (280,411) außenseitig angeformte, parallel zu einer Einsteckrichtung (E) des Verschlusselementes (28,41) ausgerichtete Stege ausgebildet sind.

8. Zerstäuber (1) nach einem der Ansprüche 1 bis 4 **dadurch gekennzeichnet, dass** das Verschlusselement (34, 37, 38) mindestens ein Spreizelement (342, 371, 382) aufweist, das radial nach außen gespreizt innen am Zerstäuber (1) anliegt, insbesondere wobei die Spreizwirkung des Spreizelements (371) aufgehoben werden kann.

9. Zerstäuber (1) nach Anspruch 1, **dadurch gekennzeichnet, dass** das Verschlusselement (45,46) mit dem Zerstäuber (1) verklebt ist.

10. Zerstäuber (1) nach Anspruch 1, **dadurch gekennzeichnet, dass** das Verschlusselement (44) mit dem Zerstäuber (1) nach Art einer Bajonettverbindung (270,440) verbunden ist.

11. Zerstäuber (1) nach einem der vorherigen Ansprüche, **dadurch gekennzeichnet, dass** das Verschlusselement (25,28-38,40-45) mit einer planen Standfläche (SF) versehen ist.

12. Zerstäuber (1) nach Anspruch 11, **dadurch gekennzeichnet, dass** das Verschlusselement (28, 29, 30) im Bereich der Standfläche (SF) wenigstens eine schlitzartige Materialausnehmung (282, 290, 302) aufweist, derart, dass **dadurch** wenigstens ein ausklappbares Greifelement (284, 291, 303) ausgebildet ist.

13. Zerstäuber (1) nach Anspruch 12, **dadurch gekennzeichnet, dass** die Materialausnehmung (282, 290, 302) teilkreisartig, insbesondere halbkreisartig ausgebildet ist.

14. Montageeinheit (1,3), bestehend aus einem Zerstäuber (1) nach einem der Ansprüche 1 bis 13 und wenigstens einem mit dem Zerstäuber (1) lösbar verbindbaren, gehäuseartigen Teil (18), an dem der Behälter (3) gehalten ist.

15. Montageeinheit (1,3) nach Anspruch 14, **dadurch gekennzeichnet, dass** Kodiermittel (26, 260 und 180,181) am Zerstäuber (1) und am gehäuseartigen Teil (18) vorhanden sind, derart, dass das gehäuseartige Teil (18) mitsamt Behälter (3) nur dann in den Zerstäuber (1) einsetzbar oder mit diesem verwendbar ist, wenn die Kodiermittel (26,260 und 180,181) eine zueinander passende Kodierung aufweisen.
